# EUROPEAN PATENT SPECIFICATION

(11) **EP 3 509 553 B1**
(45) Date of publication and mention of the grant of the patent: **14.06.2023**
(21) Application number: 17768581.5
(22) Date of filing: 06.09.2017
(51) Int. Cl.: A61F 13/511, A61F 13/512

(54) **THREE-DIMENSIONAL APERTURED SUBSTRATES**
DREIDIMENSIONALE SUBSTRATE MIT ÖFFNUNGEN
SUBSTRATS PERFORÉS TRIDIMENSIONNELS

(30) Priority: 09.09.2016 US 201662385397 P
(43) Date of publication of application: 17.07.2019
(73) Proprietor: The Procter & Gamble Company, Cincinnati, OH 45202 (US)
(72) Inventor: ROSATI, Rodrigo, 65824 Schwalbach am Taunus (DE); ERDEM, Gueltekin, Beijing 101312 (CN); KREISEL, Sascha, 65824 Schwalbach am Taunus (DE); AVILES, Misael Omar, Cincinnati, Ohio 45202 (US)
(74) Representative: P&G Patent Germany
(86) International application number: PCT/US2017/050199
(87) International publication number: WO 2018/048845

(56) References cited:
- WO-A1-2009/062998
- WO-A1-2015/015417
- US-A1- 2015 250 662
- US-B2- 9 237 973

## Description

### FIELD

The present disclosure is generally related to three-dimensional apertured substrates.

### BACKGROUND

Absorbent articles for personal hygiene, such as disposable diapers for infants, training pants for toddlers, adult incontinence undergarments, and/or sanitary napkins are designed to absorb and contain bodily exudates, in particular large quantities of urine, runny BM, and/or menses (together the "fluids" or "fluid"). These absorbent articles may comprise several layers providing different functions, for example, a topsheet, a backsheet, and an absorbent core disposed between, or partially between, the topsheet and the backsheet, among other layers, such as an acquisition and/or distribution system, if desired.

Topsheets are generally liquid permeable and are configured to receive fluids being excreted from the body and aid in directing the fluids toward an acquisition and/or distribution system and/or towards the absorbent core. Fully hydrophilic topsheets are sometimes desirable as they quickly absorb the fluids. One issue, however, with fully hydrophilic topsheets is that they may retain a good deal of the fluids owing to their hydrophilic nature. This may cause wearers of absorbent articles to feel wetness against their skin. Fully hydrophobic topsheets are sometimes desired as they do not retain the fluids. However, in fully hydrophobic topsheets, apertures are usually provided so that the fluids can be channeled through the apertures. If the effective aperture areas are too small (e.g., 1.8mm²), fluid will not enter and flow through the apertures. Thus, very large effective apertures areas (e.g., 4.6mm²) are required in related art fully hydrophobic topsheets. Larger holes, however, lead to increased rewet. Fully hydrophobic topsheets, while desirable since they do not retain fluids, have the potential risk of run-off and leakage problems, especially with smaller effective aperture areas. Three-dimensional topsheets are sometimes desirable to allow fluids to move away from a wearer's skin until the fluids may be absorbed into the absorbent article and reduce fluid/skin contact time.

To solve the problems of both fully hydrophilic topsheets and fully hydrophobic topsheets, leave the wearer's skin dry, reduce fluid/skin contact times, allow for smaller apertures and reduced skin marking, and reduce run-off and leakage problems, three-dimensional apertured topsheets should be further improved.

US 2015/250662 relates to a liquid permeable substrate for an absorbent article. The substrate includes a first layer including a hydrophobic material and a second layer including a hydrophilic material. The first layer is joined to the second layer. The substrate includes a plurality of recesses, a plurality of projections, and a plurality of land areas. The plurality of recesses, the plurality of projections, and the plurality of land areas together form a first three-dimensional surface on a first side of the substrate and a second three-dimensional surface on a second side of the substrate. The substrate has an overall z-directional height in the range of about 1000 µm to about 6000 µm according to the Overall Substrate Height Test.

WO 2015/015417 discloses a bodyside liner material for use in disposable absorbent personal care products. The liner has an average material plane and includes a plurality of raised areas extending in a z-direction from the average material plane; a plurality of apertures through the liner material; a hydrophobic treatment agent disposed on the raised areas; and a plurality of land areas disposed between the raised areas, wherein the land areas are hydrophilic. A plurality of depressed areas alternate with the plurality of raised areas, the depressed areas extending in the opposite z-direction from the average material plane, wherein the depressed areas are hydrophilic. A plurality of apertures are provided, wherein the apertures are disposed only in the depressions. A hydrophobic treatment agent disposed on the raised areas.

US 9 237 973 is concerned with personal care article comprising a nonwoven fluid permeable topsheet comprising apertured holes wherein at least 10% of the aperture holes are treated with a hydrophilic treatment agent.

WO 2009/062998 relates to a perforated material, in which the material adjacent to the holes and forming their walls is treated with a surfactant to create a surface tension gradient in the holes.

### SUMMARY

The present disclosure is generally related, in part, to three-dimensional, nonwoven, apertured substrates that are used as topsheets of absorbent articles, or that form portions of, or all of, the topsheets. The three-dimensional apertured substrates may be liquid permeable substrates. The three-dimensional apertured substrates of the present disclosure may reduce fluid/skin contact time by providing first elements having a first z-directional height and at least second elements having a second z-directional height. The first z-directional height may generally be higher than the second z-directional height. Such a structure creates a substrate having a plurality of heights. These three-dimensional apertured substrates may allow fluids, during a urination event, for example, to be received onto the substrate and moved into the second elements having the second z-directional height (lower) and/or into and through the apertures to at least reduce the amount of fluid in contact with the skin and/or to at least reduce the fluid/skin contact time. Stated another way, the first elements having the first z-directional height (higher) may be in contact with the skin, while the fluids moves via gravity into the second elements having the second z-directional height (lower height) and/or into and through the apertures. Upon information and belief, such three-dimensional structures reduce the amount of fluid on skin, give the wearer a drier, more comfortable feel, and/or reduce the pendency of fluid/skin contact. The first elements having the first z-directional height (higher) essentially serve to provide a spacer between the skin and the fluids while the substrates are channeling the fluids into the acquisition and/or distribution system and/or the absorbent core.

These three-dimensional apertured substrates (e.g., used as topsheets) comprise hydrophobic materials, such as hydrophobic nonwoven materials, but hydrophilic materials are provided only around perimeters of the apertures, only in areas closely surrounding the apertures, and/or only around perimeters of the apertures and in portions of the recesses, (together referred to herein as "proximate to the apertures"). By providing three-dimensional apertured, hydrophobic substrates, with hydrophilic materials proximate to the apertures, fluids may be quickly wicked through the apertures. The apertures now may be reduced in effective aperture area (e.g., 1.8mm²) compared to conventional hydrophobic topsheets with larger effective aperture areas (e.g., 4.6mm²), thereby reducing skin marking and rewet, while achieving about the same run-off and leakage benefits as the fully hydrophobic topsheets with the larger apertures.

Thus, the three-dimensional, nonwoven, apertured substrates or topsheets of the present disclosure solve the problems of both fully hydrophilic topsheets and fully hydrophobic topsheets, leave the wearer's skin dry, reduce fluid/skin contact, allow for smaller apertures and reduced skin marking, and reduce run-off and leakage problems.

The invention is defined by the liquid permeable nonwoven topsheet of claim 1 and the absorbent article of claim 14.

### BRIEF DESCRIPTION OF THE DRAWINGS

The above-mentioned and other features and advantages of the present disclosure, and the manner of attaining them, will become more apparent and the disclosure itself will be better understood by reference to the following description of non-limiting forms of the disclosure taken in conjunction with the accompanying drawings, wherein:
Fig. 1 is a top view of an absorbent article, wearer-facing surface facing the viewer, with some layers partially removed in accordance with the present disclosure;
Fig. 2 is a cross-sectional view of the absorbent article taken about line 2-2 of Fig. 1 in accordance with the present disclosure;
Fig. 3 is a cross-sectional view of the absorbent article taken about line 2-2 of Fig. 2 where the absorbent article has been loaded with fluid in accordance with the present disclosure;
Fig. 4 is a top view of another absorbent article, wearer-facing surface facing the viewer, with some layers partially removed in accordance with the present disclosure;
Fig. 5 is a cross-sectional view of the absorbent article taken about line 5-5 of Fig. 4 in accordance with the present disclosure;
Fig. 6 is a top view of an absorbent core of the absorbent article of Fig. 4 with some layers partially removed in accordance the present disclosure;
Fig. 7 is a cross-sectional view of the absorbent core taken about line 7-7 of Fig. 6 in accordance with the present disclosure;
Fig. 8 is a cross-sectional view of the absorbent core taken about line 8-8 of Fig. 6 in accordance with the present disclosure;
Fig. 9 is a top view of an absorbent article, wearer-facing surface facing the viewer, that is a sanitary napkin with some of the layers cut away in accordance with the present disclosure;
Fig. 10 is a top view of an absorbent article, wearer-facing surface facing the viewer, that comprises a three-dimensional, liquid permeable substrate in accordance with the present disclosure;
Fig. 11 is a perspective view of an absorbent article of Fig. 10 in accordance with the present disclosure;
Fig. 12 is an enlarged top view of a portion of the liquid permeable substrate of Fig. 10 in accordance with the present disclosure;
Fig. 13 is another enlarged top view of a portion of the liquid permeable substrate of Fig. 10 in accordance with the present disclosure;
Fig. 14 is a schematic illustration of a three-dimensional, liquid permeable substrate positioned on and/or joined to a topsheet for an absorbent article in accordance with the present disclosure;
Fig. 15 is another schematic illustration of a three-dimensional, liquid permeable substrate positioned on and/or joined to a topsheet for an absorbent article in accordance with the present disclosure;
Fig. 16 is another schematic illustration of a three-dimensional, liquid permeable substrate positioned on and/or joined to a topsheet for an absorbent article in accordance with the present disclosure;
Fig. 17 is a front view of a portion of a three-dimensional, liquid permeable substrate, wearer-facing surface facing the viewer in accordance with the present disclosure;
Fig. 18 is a front perspective view of the portion of the three-dimensional, liquid permeable substrate of Fig. 17 in accordance with the present disclosure;
Fig. 19 is another front view of a portion of a three-dimensional, liquid permeable substrate, wearer-facing surface facing the viewer in accordance with the present disclosure;
Fig. 20 is a front perspective view of the portion of the liquid permeable substrate of Fig. 19 in accordance with the present disclosure;
Fig. 21 is a back view of a portion of a three-dimensional, liquid permeable substrate, wearer-facing surface facing the viewer in accordance with the present disclosure;
Fig. 22 is a back perspective view of the portion of the three-dimensional, liquid permeable substrate of Fig. 21 in accordance with the present disclosure;
Fig. 23 is another back view of a portion of a three-dimensional, liquid permeable substrate, wearer-facing surface facing the viewer in accordance with the present disclosure;
Fig. 24 is a back perspective view of the portion of the liquid permeable substrate of Fig. 23 in accordance with the present disclosure;
Fig. 25 is a cross-sectional view of the liquid permeable substrate in accordance with the present disclosure;
Fig. 26 is a schematic illustration of one example process for forming the substrates of the present disclosure;
Fig. 27 is a view of intermeshing engagement of portions of first and second rolls in accordance with the present disclosure;
Fig. 28 is a view of a portion of the first roll in accordance with the present disclosure;
Fig. 29 is a view of a portion of the second roll in accordance with the present disclosure;
Fig. 30 is a side view of a package of absorbent articles in accordance with the present disclosure. The outer surface is illustrated as transparent for purposes of clarity.
Fig. 31 is a schematic cross-sectional illustration of an example three-dimensional, hydrophobic, nonwoven, apertured material with hydrophilic material positioned proximate to apertures in accordance with the present disclosure;
Fig. 32 is a top perspective view photograph of an example three-dimensional, hydrophobic, nonwoven, apertured material with hydrophilic material positioned proximate to apertures in accordance with the present disclosure;
Fig. 33 is a bottom perspective view photograph of an example three-dimensional, hydrophobic, nonwoven, apertured material with hydrophilic material positioned proximate to apertures in accordance with the present disclosure;
Fig. 34 is a schematic illustration of a hydrophilic material being applied to a three-dimensional, nonwoven, hydrophobic, apertured, material proximate to the apertures in accordance with the present disclosure;
Fig. 35 is a schematic illustration of a hydrophilic material being applied to a three-dimensional, nonwoven, hydrophobic, apertured, material proximate to the apertures in accordance with the present disclosure;
Fig. 36 is a schematic cross-sectional illustration of an example three-dimensional, hydrophilic, nonwoven, apertured material with a hydrophobic material positioned on the projections and hydrophilic material proximate to the apertures in accordance with the present disclosure; and
Fig. 37 is a schematic illustration of a hydrophobic material being applied to a three-dimensional, nonwoven, hydrophilic, apertured, material on the projections in accordance with the present disclosure.

### DETAILED DESCRIPTION

Various non-limiting forms of the present disclosure will now be described to provide an overall understanding of the principles of the structure, function, manufacture, and use of the three-dimensional apertured substrates disclosed herein. One or more examples of these non-limiting embodiments are illustrated in the accompanying drawings. Those of ordinary skill in the art will understand that the three-dimensional apertured substrates described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

### Introduction

As used herein, the term "absorbent article" refers to disposable devices such as pre-mature infant, infant, child, or adult diapers, adult incontinence products, training pants, sanitary napkins, and the like which are placed against or in proximity to a body of a wearer to absorb and contain the various fluids discharged from the body. Typically, these absorbent articles comprise a topsheet, backsheet, an absorbent core, optionally an acquisition system and/or a distribution system (which may be comprised of one or several layers), and typically other components, with the absorbent core normally placed at least partially between the backsheet and the acquisition and/or distribution system or between the topsheet and the backsheet. The absorbent articles comprising three-dimensional, apertured liquid permeable substrates of the present disclosure will be further illustrated in the below description and in the Figures in the form of one or more components of taped diaper. Nothing in this description should be, however, considered limiting the scope of the claims. As such the present disclosure applies to any suitable form of absorbent articles (e.g., diapers, training pants, adult incontinence products, sanitary napkins).

As used herein, the term "nonwoven web" means a manufactured sheet, web, or batt of directionally or randomly orientated fibers, bonded by friction, and/or cohesion, and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers may have diameters ranging from less than about 0.001 mm to more than about 0.2 mm and may come in several different forms such as short fibers (known as staple, or chopped), continuous single fibers (filaments or monofilaments), untwisted bundles of continuous filaments (tow), and twisted bundles of continuous filaments (yam). Nonwoven webs may be formed by many processes such as meltblowing, spunbonding, solvent spinning, electrospinning, carding, and airlaying. The basis weight of nonwoven webs is usually expressed in grams per square meter (g/m² or gsm).

As used herein, the terms "joined", "bonded", or "attached" encompasses configurations wherein an element is directly secured to another element by affixing the element directly to the other element, and configurations whereby an element is indirectly secured to another element by affixing the element to intermediate member(s) which in turn are affixed to the other element.

As used herein, the term "machine direction" or "MD" is the direction that is substantially parallel to the direction of travel of a substrate as it is made. The "cross direction" or "CD" is the direction substantially perpendicular to the MD and in the plane generally defined by the substrate.

As used herein, the term "hydrophilic", refers to a material having a contact angle less than or equal to 90° according to The American Chemical Society Publication "Contact Angle, Wettability, and Adhesion," edited by Robert F. Gould and copyrighted in 1964.

As used herein, the term "hydrophobic", refers to a material or layer having a contact angle greater than or equal to 90° according to The American Chemical Society Publication "Contact Angle, Wettability, and Adhesion," edited by Robert F. Gould and copyrighted in 1964.

### General Description of the Absorbent Article

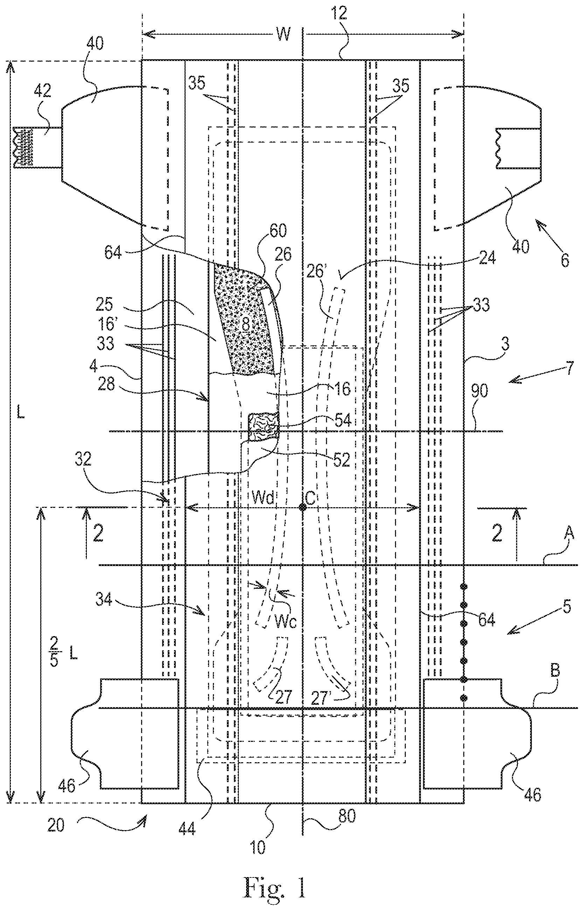

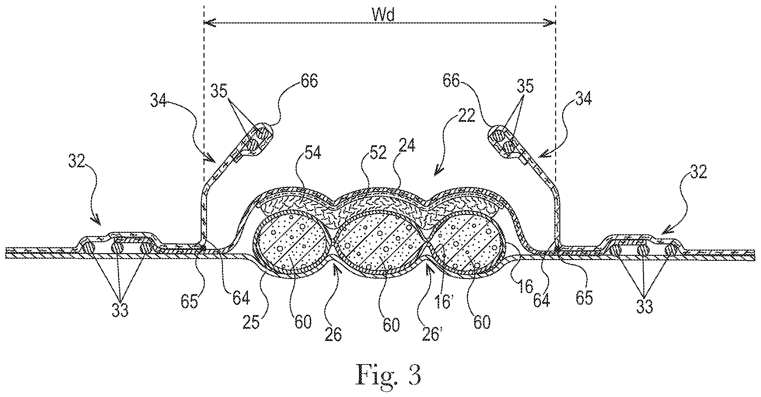

An example absorbent article in the form of a diaper 20 is represented in Figs. 1-3. Fig. 1 is a plan view of the example diaper 20, in a flat-out state, with portions of the structure being cut-away to more clearly show the construction of the diaper 20. The wearer-facing surface of the diaper 20 of Fig. 1 is facing the viewer. This diaper 20 is shown for illustration purpose only as the three-dimensional substrates of the present disclosure may be used as one or more components of an absorbent article.

The absorbent article 20 may comprise a liquid permeable topsheet 24, a liquid impermeable backsheet 25, an absorbent core 28 positioned at least partially intermediate the topsheet 24 and the backsheet 25, and barrier leg cuffs 34. The absorbent article may also comprise an acquisition and/or distribution system ("ADS") 50, which in the example represented comprises a distribution layer 54 and an acquisition layer 52, which will be further detailed below. The absorbent article may also comprise elasticized gasketing cuffs 32 comprising elastics 33 joined to a chassis of the absorbent article, typically via the topsheet and/or backsheet, and substantially planar with the chassis of the diaper.

The figures also show typical taped diaper components such as a fastening system comprising tabs 42 attached towards the rear edge of the article and cooperating with a landing zone 44 on the front of the absorbent article. The absorbent article may also comprise other typical elements, which are not represented, such as a rear elastic waist feature, a front elastic waist feature, transverse barrier cuff(s), and/or a lotion application, for example.

The absorbent article 20 comprises a front waist edge 10, a rear waist edge 12 longitudinally opposing the front waist edge 10, a first side edge 3, and a second side edge 4 laterally opposing the first side edge 3. The front waist edge 10 is the edge of the article which is intended to be placed towards the front of the user when worn, and the rear waist edge 12 is the opposite edge. The absorbent article may have a longitudinal axis 80 extending from the lateral midpoint of the front waist edge 10 to a lateral midpoint of the rear waist edge 12 of the article and dividing the article in two substantially symmetrical halves relative to the longitudinal axis 80, with the article placed flat and viewed from above as in Fig. 1. The absorbent article may also have a lateral axis 90 extending from the longitudinal midpoint of the first side edge 3 to the longitudinal midpoint of the second side edge 4. The length, L, of the article may be measured along the longitudinal axis 80 from the front waist edge 10 to the rear waist edge 12. The width, W, of the article may be measured along the lateral axis 90 from the first side edge 3 to the second side edge 4. The article may comprise a crotch point C defined herein as the point placed on the longitudinal axis at a distance of two fifth (2/5) of L starting from the front edge 10 of the article 20. The article may comprise a front waist region 5, a rear waist region 6, and a crotch region 7. The front waist region 5, the rear waist region 6, and the crotch region 7 each define 1/3 of the longitudinal length, L, of the absorbent article.

The absorbent core 28 may comprise an absorbent material comprising at least 75%, at least 80%, at least 90%, at least 95%, or at least 99% by weight of absorbent material and a core wrap enclosing the superabsorbent polymers. The core wrap may typically comprise two materials, substrates, or nonwoven materials 16 and 16' for the top side and bottom side of the core. The core may comprises one or more channels, represented in Fig. 1 as the four channels 26, 26' and 27, 27'. The channels 26, 26', 27 and 27' are optional features. Instead, the core may not have any channels or may have any number of channels. The channels may be embossed into the absorbent material or the absorbent core.

These and other components of the example absorbent article will now be discussed in more details.

### Topsheet

In the present disclosure, the topsheet (the portion of the absorbent article that contacts the wearer's skin and receives the fluids) may be formed of a portion of, or all of, one or more of the three-dimensional, apertured substrates described herein and/or have one or more three-dimensional, apertured substrates positioned thereon and/or joined thereto, so that the three-dimensional, apertured substrate(s) contact(s) the wearer's skin. Other portions of the topsheet (other than the three-dimensional, apertured substrates) may also contact the wearer's skin. A typical topsheet is described below, although it will be understood that this topsheet 24, or portions thereof, may be replaced by the three-dimensional, apertured, nonwoven, substrates described herein. In some instances, the three-dimensional, apertured, nonwoven substrates may be positioned as a strip or a patch on top of the typical topsheet 24, as is described herein. In another instance, the three-dimensional apertured, nonwoven substrates may form a central portion of the topsheet, while a flat nonwoven material may forms the side portions.

The topsheet 24 may be the part of the absorbent article that is in contact with the wearer's skin. The topsheet 24 may be joined to the backsheet 25, the core 28 and/or any other layers as is known to those of skill in the art. Usually, the topsheet 24 and the backsheet 25 are joined directly to each other in some locations (e.g., on or close to the periphery of the absorbent article) and are indirectly joined together in other locations by directly joining them to one or more other elements of the article 20.

The topsheet 24 may be compliant, soft-feeling, and non-irritating to the wearer's skin. Further, a portion of, or all of, the topsheet 24 may be liquid permeable, permitting liquids to readily penetrate through its thickness.

The three-dimensional, apertured nonwoven substrates, used as topsheets, are further discussed herein.

### Backsheet

The backsheet 25 is generally that portion of the absorbent article 20 positioned adjacent the garment-facing surface of the absorbent core 28 and which prevents, or at least inhibits, the fluids absorbed and contained in the absorbent core from soiling articles such as bedsheets and undergarments. The backsheet 25 is typically impermeable, or at least substantially impermeable, to fluids (e.g., urine). Other suitable backsheet materials may include breathable materials which permit vapors to escape from the absorbent article 20 while still preventing, or at least inhibiting, fluids from passing through the backsheet 25.

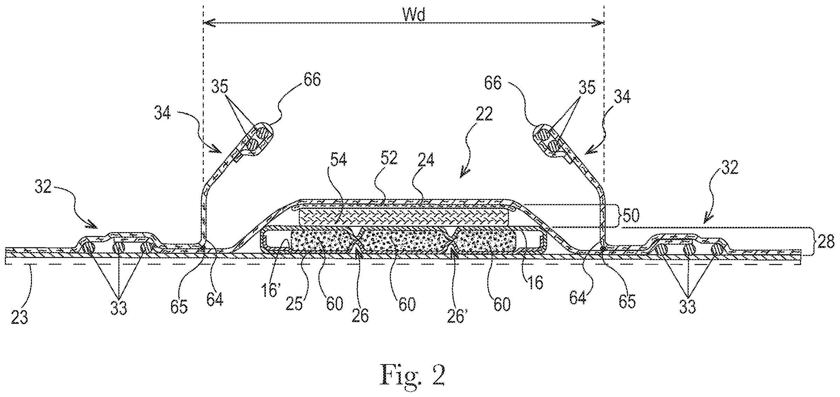

An outer cover 23 may cover at least a portion of, or all of, the backsheet 25 to form a soft garment-facing surface of the absorbent article. The outer cover 23 may be formed of one or more nonwoven materials or other suitable materials. The outer cover 23 is illustrated in dash in Fig. 2, as an example.

### Absorbent Core

As used herein, the term "absorbent core" refers to the component of the absorbent article having the most absorbent capacity and comprising an absorbent material and a core wrap or core bag enclosing the absorbent material. The term "absorbent core" does not include the acquisition and/or distribution system or any other components of the article which are not either integral part of the core wrap or core bag or placed within the core wrap or core bag. The absorbent core may comprise, consist essentially of, or consist of, a core wrap, an absorbent material (e.g., superabsorbent polymers) as discussed, and one or more adhesives.

The absorbent core 28 may comprise an absorbent material with a high amount of superabsorbent polymers (herein abbreviated as "SAP") enclosed within the core wrap. The SAP content may represent 70% to 100% or at least 70%, at least 75%, at least 80%, at least 85%, at least 90%, at least 95%, at least 99%, or 100%, by weight of the absorbent material, enclosed in the core wrap. The core wrap is not considered as absorbent material for the purpose of assessing the percentage of SAP in the absorbent core. The absorbent material may in particular comprise less than 15% weight percent, less than 10% weight percent, less than 5% weight percent, less than 3% weight percent, less than 2% weight percent, less than 1% weight percent, of natural, cellulosic and/or synthetic fibers, or may even be substantially free of natural, cellulosic, and/or synthetic fibers. The core may also contain airfelt or cellulosic fibers with or without SAP.

By "absorbent material" it is meant a material which has some absorbency property or liquid retaining properties, such as SAP, cellulosic fibers, as well as synthetic fibers. Typically, glues used in making absorbent cores have no or little absorbency properties and are not considered as absorbent material.

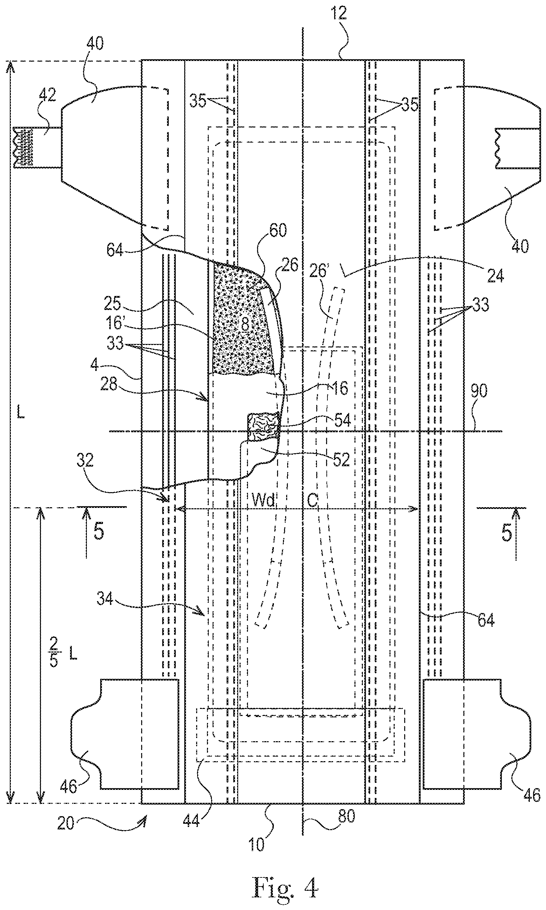

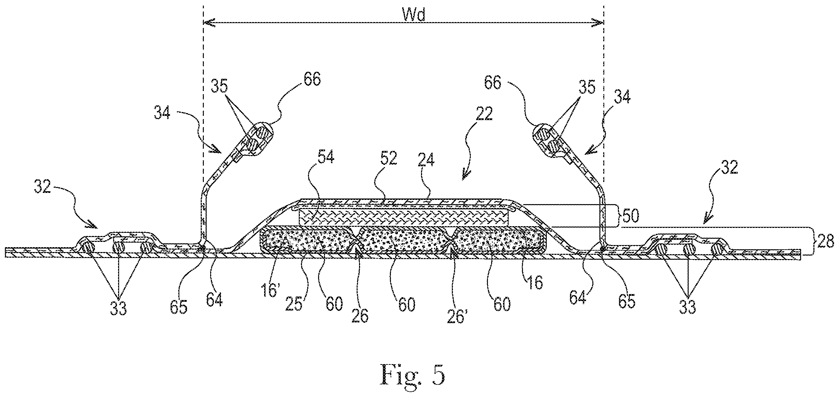

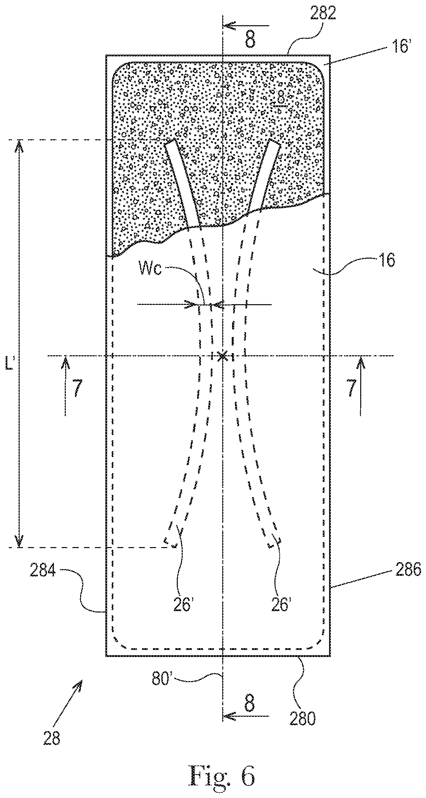

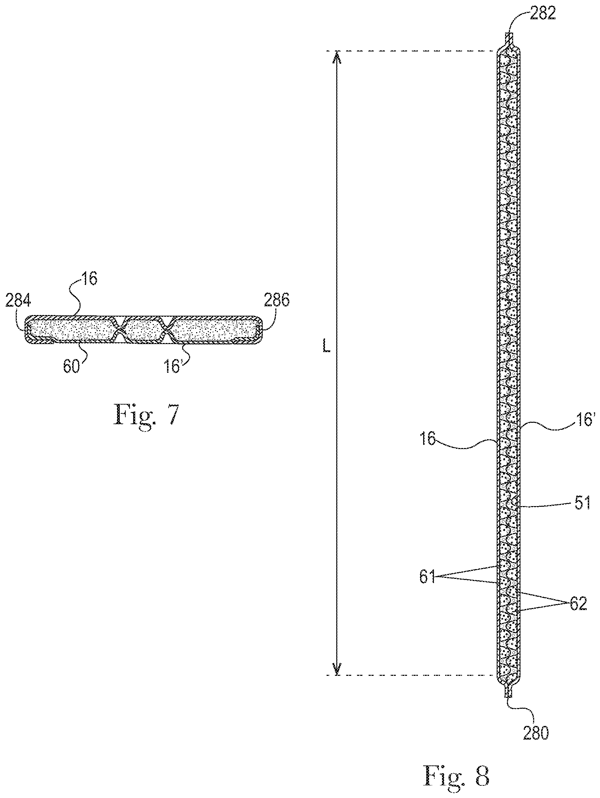

The example absorbent core 28 of the absorbent article 20 of Figs. 4-5 is shown in isolation in Figs. 6-8. The absorbent core 28 may comprises a front side 280, a rear side 282, and two longitudinal sides 284, 286 joining the front side 280 and the rear side 282. The absorbent core 28 may also comprise a generally planar top side and a generally planar bottom side. The front side 280 of the core is the side of the core intended to be placed towards the front waist edge 10 of the absorbent article. The core 28 may have a longitudinal axis 80' corresponding substantially to the longitudinal axis 80 of the absorbent article 20, as seen from the top in a planar view as in Fig. 1. The absorbent material may be distributed in higher amount towards the front side 280 than towards the rear side 282 as more absorbency may be required at the front in particular absorbent articles. The core wrap may be formed by two nonwoven materials, substrates, laminates, or other materials, 16, 16' which may be at least partially sealed along the sides 284, 286 of the absorbent core 28. The core wrap may be at least partially sealed along its front side 280, rear side 282, and two longitudinal sides 284, 286 so that substantially no absorbent material leaks out of the absorbent core wrap. The first material, substrate, or nonwoven 16 may at least partially surround the second material, substrate, or nonwoven 16' to form the core wrap, as illustrated in Fig. 7. The first material 16 may surround a portion of the second material 16' proximate to the first and second side edges 284 and 286.

The absorbent core may comprise adhesive, for example, to help immobilizing the SAP within the core wrap and/or to ensure integrity of the core wrap, in particular when the core wrap is made of two or more substrates. The adhesive may be a hot melt adhesive, supplied, by H.B. Fuller, for example. The core wrap may extend to a larger area than strictly needed for containing the absorbent material within.

Cores comprising relatively high amount of SAP with various core designs are disclosed in U.S. Pat. No. 5,599,335 (Goldman), EP 1 447 066 (Busam), WO 95/11652 (Tanzer), U.S. Pat. Publ. No. 2008/0312622 A1 (Hundorf), and WO 2012/052172 (Van Malderen).

The absorbent material may be a continuous layer present within the core wrap. Alternatively, the absorbent material may be comprised of individual pockets or stripes of absorbent material enclosed within the core wrap. In the first case, the absorbent material may be, for example, obtained by the application of a single continuous layer of absorbent material. The continuous layer of absorbent material, in particular of SAP, may also be obtained by combining two absorbent layers having discontinuous absorbent material application patterns, wherein the resulting layer is substantially continuously distributed across the absorbent particulate polymer material area, as disclosed in U.S. Pat. Appl. Pub. No. 2008/0312622 A1 (Hundorf), for example. The absorbent core 28 may comprise a first absorbent layer and a second absorbent layer. The first absorbent layer may comprise the first material 16 and a first layer 61 of absorbent material, which may be 100% or less of SAP. The second absorbent layer may comprise the second material 16' and a second layer 62 of absorbent material, which may also be 100% or less of SAP. The absorbent core 28 may also comprise a fibrous thermoplastic adhesive material 51 at least partially bonding each layer of absorbent material 61, 62 to its respective material 16 or 16'. This is illustrated in Figs. 7-8, as an example, where the first and second SAP layers have been applied as transversal stripes or "land areas" having the same width as the desired absorbent material deposition area on their respective substrate before being combined. The stripes may comprise different amounts of absorbent material (SAP) to provide a profiled basis weight along the longitudinal axis of the core 80. The first material 16 and the second material 16' may form the core wrap.

The fibrous thermoplastic adhesive material 51 may be at least partially in contact with the absorbent material 61, 62 in the land areas and at least partially in contact with the materials 16 and 16' in the junction areas. This imparts an essentially three-dimensional structure to the fibrous layer of thermoplastic adhesive material 51, which in itself is essentially a two-dimensional structure of relatively small thickness, as compared to the dimension in length and width directions. Thereby, the fibrous thermoplastic adhesive material may provide cavities to cover the absorbent material in the land areas, and thereby immobilizes this absorbent material, which may be 100% or less of SAP.

### Core Wrap

The core wrap may be made of a single substrate, material, or nonwoven folded around the absorbent material, or may comprise two (or more) substrates, materials, or nonwovens which are attached to another. Typical attachments are the so-called C-wrap and/or sandwich wrap. In a C-wrap, as illustrated, for example, in Figs. 2 and 7, the longitudinal and/or transversal edges of one of the substrates are folded over the other substrate to form flaps. These flaps are then bonded to the external surface of the other substrate, typically by gluing.

The core wrap may be at least partially sealed along all the sides of the absorbent core so that substantially no absorbent material leaks out of the core.

If the core wrap is formed by two substrates 16, 16', four seals may be used to enclose the absorbent material 60 within the core wrap. For example, a first substrate 16 may be placed on one side of the core (the top side as represented in the Figures) and extend around the core's longitudinal edges to at least partially wrap the opposed bottom side of the core. The second substrate 16' may be present between the wrapped flaps of the first substrate 16 and the absorbent material 60. The flaps of the first substrate 16 may be glued to the second substrate 16' to provide a strong seal. This so called C-wrap construction may provide benefits such as improved resistance to bursting in a wet loaded state compared to a sandwich seal. The front side and rear side of the core wrap may then also be sealed by gluing the first substrate and second substrate to another to provide complete encapsulation of the absorbent material across the whole of the periphery of the core. For the front side and rear side of the core, the first and second substrates may extend and may be joined together in a substantially planar direction, forming for these edges a so-called sandwich construction. In the so-called sandwich construction, the first and second substrates may also extend outwardly on all sides of the core and be sealed flat, or substantially flat, along the whole or parts of the periphery of the core typically by gluing and/or heat/pressure bonding. In an example, neither the first nor the second substrates need to be shaped, so that they may be rectangularly cut for ease of production but other shapes are within the scope of the present disclosure.

The core wrap may also be formed by a single substrate which may enclose as in a parcel wrap the absorbent material and be sealed along the front side and rear side of the core and one longitudinal seal.

### SAP Deposition Area

The absorbent material deposition area 8 may be defined by the periphery of the layer formed by the absorbent material 60 within the core wrap, as seen from the top side of the absorbent core. The absorbent material deposition area 8 may have various shapes, in particular, a so-called "dog bone" or "hour-glass" shape, which shows a tapering along its width towards the middle or "crotch" region of the core. In this way, the absorbent material deposition area 8 may have a relatively narrow width in an area of the core intended to be placed in the crotch region of the absorbent article, as illustrated in Fig. 1. This may provide better wearing comfort. The absorbent material deposition area 8 may also be generally rectangular, for example as shown in Figs. 4-6, but other deposition areas, such as a rectangular, "T," "Y," "sand-hour," or "dog-bone" shapes are also within the scope of the present disclosure. The absorbent material may be deposited using any suitable techniques, which may allow relatively precise deposition of SAP at relatively high speed.

### Channels

The absorbent material deposition area 8 may comprise at least one channel 26, which is at least partially oriented in the longitudinal direction of the article 80 (i.e., has a longitudinal vector component). Other channels may be at least partially oriented in the lateral direction (i.e., has a lateral vector component) or in any other direction. In the following, the plural form "channels" will be used to mean "at least one channel". The channels may have a length L' projected on the longitudinal axis 80 of the article that is at least 10% of the length L of the article. The channels may be formed in various ways. For example, the channels may be formed by zones within the absorbent material deposition area 8 which may be substantially free of, or free of, absorbent material, in particular SAP. In addition or alternatively, the channel(s) may also be formed by continuously or discontinuously bonding the top side of the core wrap to the bottom side of the core wrap through the absorbent material deposition area 8. The channels may be continuous but it is also envisioned that the channels may be intermittent. The acquisition-distribution system or layer 50, or another layer of the article, may also comprise channels, which may or not correspond to the channels of the absorbent core.

In some instances, the channels may be present at least at the same longitudinal level as the crotch point C or the lateral axis 60 in the absorbent article, as represented in Fig. 1 with the two longitudinally extending channels 26, 26'. The channels may also extend from the crotch region 7 or may be present in the front waist region 5 and/or in the rear waist region 6 of the article.

The absorbent core 28 may also comprise more than two channels, for example, at least 3, at least 4, at least 5, or at least 6 or more. Shorter channels may also be present, for example in the rear waist region 6 or the front waist region 5 of the core as represented by the pair of channels 27, 27' in Fig. 1 towards the front of the article. The channels may comprise one or more pairs of channels symmetrically arranged, or otherwise arranged relative to the longitudinal axis 80.

The channels may have a width Wc along at least part of their length which is at least 2 mm, at least 3 mm, at least 4 mm, up to for example 20 mm, 16 mm, or 12 mm, for example. The width of the channel(s) may be constant through substantially the whole length of the channel or may vary along its length. When the channels are formed by absorbent material-free zone within the absorbent material deposition area 8, the width of the channels is considered to be the width of the material free zone, disregarding the possible presence of the core wrap within the channels. If the channels are not formed by absorbent material free zones, for example mainly though bonding of the core wrap through the absorbent material zone, the width of the channels is the width of this bonding.

At least some or all of the channels may be permanent channels, meaning their integrity is at least partially maintained both in the dry state and in the wet state. Permanent channels may be obtained by provision of one or more adhesive materials, for example, the fibrous layer of adhesive material or construction glue that helps adhere a substrate with an absorbent material within the walls of the channel. Permanent channels may also be formed by bonding the upper side and lower side of the core wrap (e.g., the first substrate 16 and the second substrate 16') and/or the topsheet 24 to the backsheet 25 together through the channels. Typically, an adhesive may be used to bond both sides of the core wrap or the topsheet and the backsheet through the channels, but it is possible to bond via other known processes, such as pressure bonding, ultrasonic bonding, heat bonding, or combination thereof. The core wrap or the topsheet 24 and the backsheet 25 may be continuously bonded or intermittently bonded along the channels. The channels may advantageously remain or become visible at least through the topsheet and/or backsheet when the absorbent article is fully loaded with a fluid. This may be obtained by making the channels substantially free of SAP, so they will not swell, and sufficiently large so that they will not close when wet. Furthermore, bonding the core wrap to itself or the topsheet to the backsheet through the channels may be advantageous.

### Barrier Leg Cuffs

The absorbent article may comprise a pair of barrier leg cuffs 34. Each barrier leg cuff may be formed by a piece of material which is bonded to the article so it may extend upwards from a wearer-facing surface of the absorbent article and provide improved containment of fluids and other body exudates approximately at the junction of the torso and legs of the wearer. The barrier leg cuffs are delimited by a proximal edge 64 joined directly or indirectly to the topsheet 24 and/or the backsheet 25 and a free terminal edge 66, which is intended to contact and form a seal with the wearer's skin. The barrier leg cuffs 34 extend at least partially between the front waist edge 10 and the rear waist edge 12 of the absorbent article on opposite sides of the longitudinal axis 80 and are at least present at the level of the crotch point (C) or crotch region. The barrier leg cuffs may be joined at the proximal edge 64 with the chassis of the article by a bond 65 which may be made by gluing, fusion bonding, or a combination of other suitable bonding processes. The bond 65 at the proximal edge 64 may be continuous or intermittent. The bond 65 closest to the raised section of the leg cuffs delimits the proximal edge 64 of the standing up section of the leg cuffs.

The barrier leg cuffs may be integral with the topsheet 24 or the backsheet 25 or may be a separate material joined to the article's chassis. Each barrier leg cuff 34 may comprise one, two or more elastic strings 35 close to the free terminal edge 66 to provide a better seal.

In addition to the barrier leg cuffs 34, the article may comprise gasketing cuffs 32, which are joined to the chassis of the absorbent article, in particular to the topsheet 24 and/or the backsheet 25 and are placed externally relative to the barrier leg cuffs. The gasketing cuffs 32 may provide a better seal around the thighs of the wearer. Each gasketing leg cuff may comprise one or more elastic strings or elastic elements 33 in the chassis of the absorbent article between the topsheet 24 and backsheet 25 in the area of the leg openings.

### Acquisition-Distribution System

The absorbent articles of the present disclosure may comprise an acquisition-distribution layer or system 50 ("ADS"). One function of the ADS is to quickly acquire one or more of the fluids and distribute them to the absorbent core in an efficient manner. The ADS may comprise one, two or more layers, which may form a unitary layer or may remain as discrete layers which may be attached to each other. In an example, the ADS may comprise two layers: a distribution layer 54 and an acquisition layer 52 disposed between the absorbent core and the topsheet, but the present disclosure is not so limited.

In one example, the ADS may not be provided, or only one layer of the ADS may be provided, such as the distribution layer only or the acquisition layer only. When one of the three-dimensional, liquid permeable, apertured substrates of the present disclosure is used as a portion of, or all of, a topsheet, or positioned on a topsheet, dryness performance of the liquid permeable substrates may be improved if only one or no layers of the ADS are present. This is owing to the fact that fluids are easily able to wick through the liquid permeable substrates directly into the absorbent core 28 and/or into one layer of the ADS.

### Distribution Layer

The distribution layer of the ADS may comprise at least 50% by weight of cross-linked cellulose fibers. The cross-linked cellulosic fibers may be crimped, twisted, or curled, or a combination thereof including crimped, twisted, and curled. This type of material is disclosed in U.S. Pat. Publ. No. 2008/0312622 A1 (Hundorf).

### Acquisition Layer

The ADS 50 may comprise an acquisition layer 52. The acquisition layer may be disposed between the distribution layer 54 and the topsheet 24. The acquisition layer 52 may be or may comprise a nonwoven material, such as a hydrophilic SMS or SMMS material, comprising a spunbonded, a melt-blown and a further spunbonded layer or alternatively a carded staple fiber chemical-bonded nonwoven. The nonwoven material may be latex bonded.

### Fastening System

The absorbent article may include a fastening system. The fastening system may be used to provide lateral tensions about the circumference of the absorbent article to hold the absorbent article on the wearer as is typical for taped diapers. This fastening system may not be necessary for training pant articles since the waist region of these articles is already bonded. The fastening system may comprise a fastener such as tape tabs, hook and loop fastening components, interlocking fasteners such as tabs & slots, buckles, buttons, snaps, and/or hermaphroditic fastening components, although any other suitable fastening mechanisms are also within the scope of the present disclosure. A landing zone 44 is normally provided on the garment-facing surface of the front waist region 5 for the fastener to be releasably attached thereto. The landing zone 44 may merely be a portion of the outer cover nonwoven material in the front waist region 5 or may be a separate component attached to the outer cover nonwoven material.

### Front and Rear Ears

The absorbent article may comprise front ears 46 and rear ears 40. The ears may be an integral part of the chassis, such as formed from the topsheet 24 and/or backsheet 26 as side panels. Alternatively, as represented on Fig. 1, the ears may be separate elements attached by gluing, heat embossing, and/or pressure bonding. The rear ears 40 may be stretchable to facilitate the attachment of the tabs 42 to the landing zone 44 and maintain the taped diapers in place around the wearer's waist. The rear ears 40 may also be elastic or extensible to provide a more comfortable and contouring fit by initially conformably fitting the absorbent article to the wearer and sustaining this fit throughout the time of wear well past when absorbent article has been loaded with fluids since the elasticized ears allow the sides of the absorbent article to expand and contract.

### Relations Between the Layers

Typically, adjacent layers and components may be joined together using conventional bonding methods, such as adhesive coating via slot coating or spraying on the whole or part of the surface of the layer, thermo-bonding, pressure bonding, or combinations thereof. This bonding is not represented in the Figures (except for the bonding between the raised element of the leg cuffs 65 with the topsheet 24) for clarity and readability, but bonding between the layers of the article should be considered to be present unless specifically excluded. Adhesives may be used to improve the adhesion of the different layers between the backsheet 25 and the core wrap. The glue may be any suitable hotmelt glue known in the art.

### Sanitary Napkin

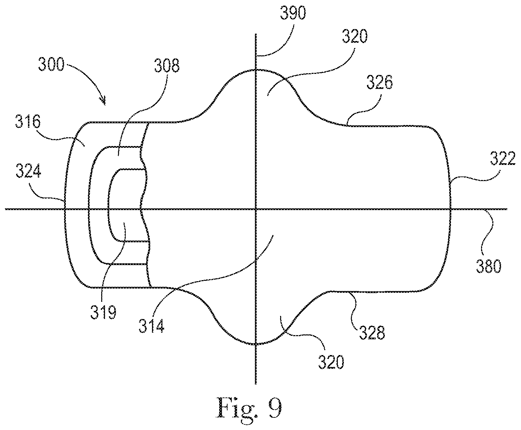

The three-dimensional, nonwoven, apertured, substrates of the present disclosure may form a portion of a topsheet, form the topsheet, form a portion of, or all of a secondary topsheet, or be positioned on or joined to at least a portion of the topsheet of a sanitary napkin. Referring to Fig. 9, the absorbent article may comprise a sanitary napkin 300. The sanitary napkin 300 may comprise a liquid permeable topsheet 314, a liquid impermeable, or substantially liquid impermeable, backsheet 316, and an absorbent core 308. The absorbent core 308 may have any or all of the features described herein with respect to the absorbent cores 28 and, in some forms, may have a secondary topsheet instead of the acquisition-distribution system disclosed above. The sanitary napkin 300 may also comprise wings 320 extending outwardly with respect to a longitudinal axis 380 of the sanitary napkin 300. The sanitary napkin 300 may also comprise a lateral axis 390. The wings 320 may be joined to the topsheet 314, the backsheet 316, and/or the absorbent core 308. The sanitary napkin 300 may also comprise a front edge 322, a rear edge 324 longitudinally opposing the front edge 322, a first side edge 326, and a second side edge 328 longitudinally opposing the first side edge 326. The longitudinal axis 380 may extend from a midpoint of the front edge 322 to a midpoint of the rear edge 324. The lateral axis 390 may extend from a midpoint of the first side edge 326 to a midpoint of the second side edge 328. The sanitary napkin 300 may also be provided with additional features commonly found in sanitary napkins as is generally known in the art, such as a secondary topsheet 319, for example.

### Three-Dimensional, Apertured Substrates

The three-dimensional, apertured liquid permeable substrates (e.g., topsheets) of the present disclosure may comprise first elements (e.g., projections) that have a first z-directional height and at least second elements (e.g., land areas) that have a second z-directional height. The substrates may also have a plurality of apertures. The substrates may also have at least third elements having at least a third z-directional height. Owing to such structures, fluids may be quickly moved away from the skin of a wearer, leaving primarily the first elements having the first z-directional heights contacting the skin of the wearer, thereby making the wearer feel dryer. The fluids may flow via gravity or via capillary gradient into the second elements and/or at least third elements into and through the apertures, so that the fluids may be absorbed into the absorbent articles. By providing the three-dimensional, apertured substrates of the present disclosure, fluid/skin contact and the time that fluids are in contact with the skin of a wearer may be reduced. Further, the first elements having the first z-directional heights may act as a spacer between the fluids and the skin of the wearer while the fluids are being absorbed into the absorbent article. The substrates may comprise two hydrophobic nonwoven layers with a hydrophilic material positioned proximate to the apertures as described further herein. In other instances, the substrates may comprise a single hydrophobic nonwoven layer or three hydrophobic nonwoven layers.

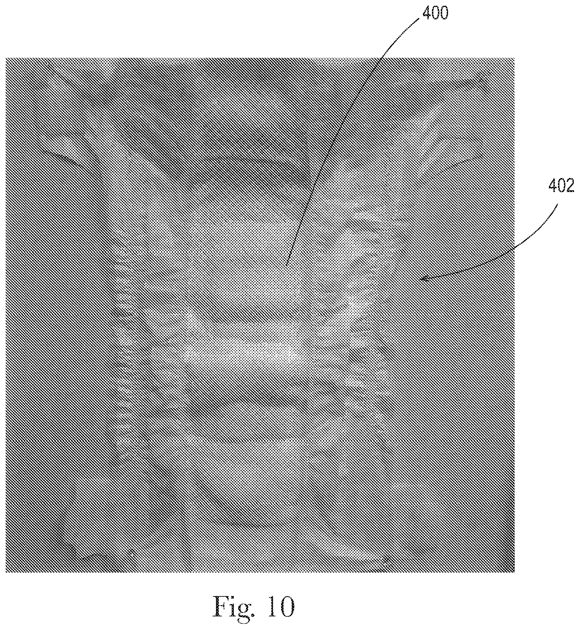

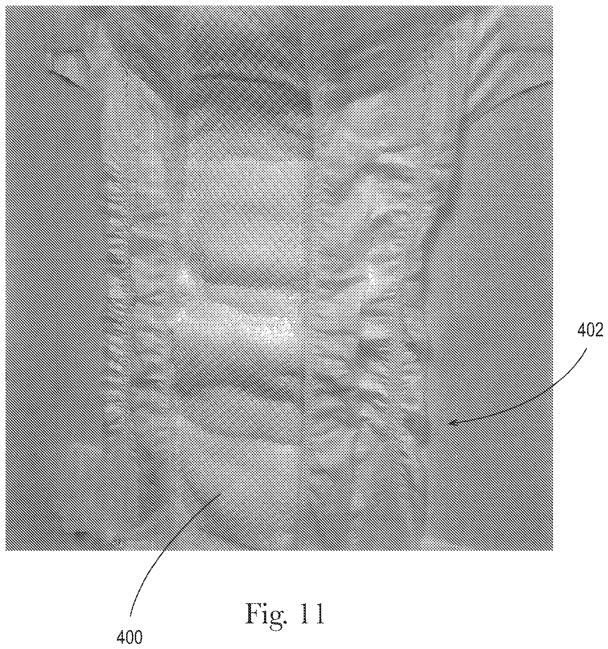

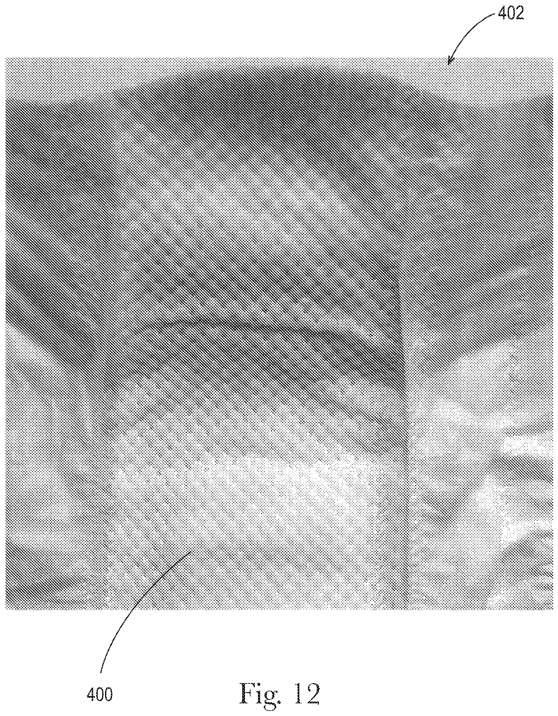

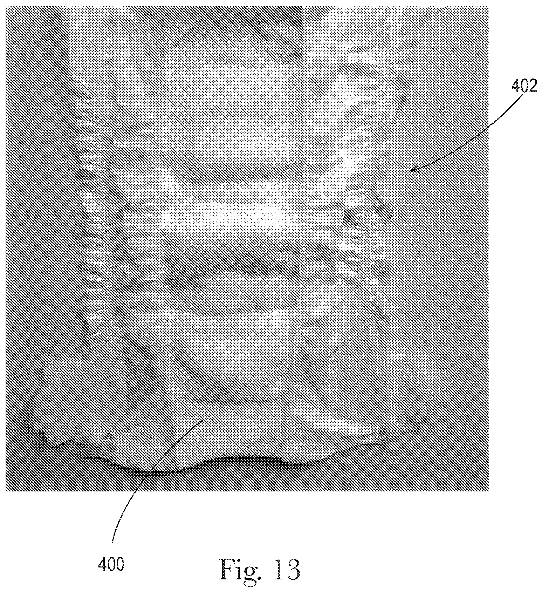

Referring to Figs. 10-13, a three-dimensional, apertured, liquid permeable substrate 400 is illustrated as of a topsheet of an absorbent article 402. Fig. 10 is a top view of the absorbent article 402 with the wearer-facing surface facing the viewer. Fig. 11 is a perspective view of the absorbent article 402 with the wearer-facing surface facing the viewer. Fig. 12 is a top view of a portion of the liquid permeable, apertured substrate 400 on the absorbent article with the wearer-facing surface facing the viewer. Fig. 13 is another top view of a portion of the three-dimensional, apertured substrate 400 on the absorbent article 402 with the wearer-facing surface facing the viewer.

In one form, the three-dimensional, apertured substrate 400, or other three-dimensional, apertured substrates described herein, may comprise a patch or strip positioned on and/or joined to a topsheet of the absorbent article 402. The patch or strip may be bonded to the topsheet, adhesively attached to the topsheet, cold-pressure welded to the topsheet, ultrasonically bonded to the topsheet, and/or otherwise joined to the topsheet. Alternatively, the three-dimensional, apertured substrates of the present disclosure may comprise the topsheet (e.g., topsheet 24), form all of the topsheet, or form a portion of the topsheet. Also, the topsheet 24 may be comprised only of one or more of the three-dimensional, apertured substrates of the present disclosure. In any of the various configurations, the three-dimensional, apertured substrates of the present disclosure are intended to form at least a portion of the wearer-facing surface of an absorbent article and be in at least partial contact with the skin of a wearer.

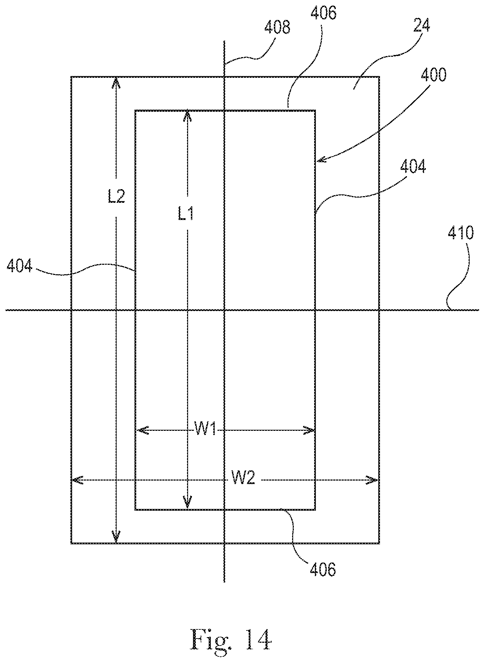

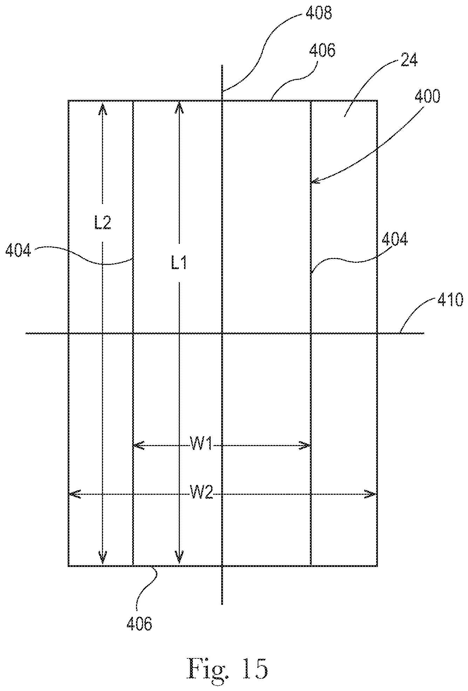

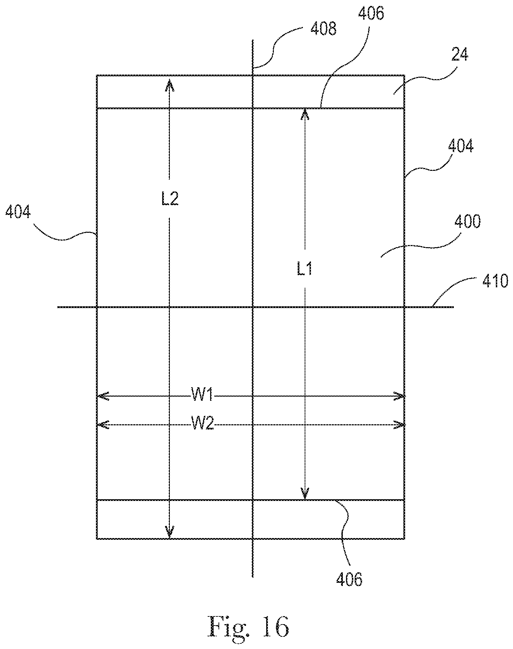

Referring to Figs. 14-16, the three-dimensional, apertured substrate 400, or other three-dimensional, apertured substrates described herein, in a patch or strip form joined to the topsheet 24, may have a cross machine directional width of W1, while the topsheet 24 may have a cross machine directional width of W2. W1 may be less than, the same as, substantially the same as, or greater than (not illustrated) the width W2. The width W1 may also vary or be constant throughout a longitudinal length of the liquid permeable substrates. Still referring to Figs. 14-16, the three-dimensional, apertured substrate 400, or other three-dimensional, apertured substrates described herein, in a patch or strip form, may have a machine directional length of L1, while the topsheet 24 may have a machine directional length of L2. L1 may be less than, the same as, substantially the same as, or greater than (not illustrated) the length L2. The length L1 may vary or be constant across the width W1 of the liquid permeable substrates. Although not illustrated in Figs. 14-16, the lengths and widths of the topsheet 24 and the three-dimensional, apertured substrates may be the same, or substantially the same.

Although the patch or strip of the three-dimensional, apertured substrate 400 is illustrated as being rectangular in Figs. 14-16, the three-dimensional, apertured substrates of the present disclosure may also have any other suitable shapes, such a front/back profiled shape (i.e., wider in the front, wider in the back, and/or narrower in the crotch), a square shape, an ovate shape, or other suitable shape. The side edges 404 and/or the end edge 406 of the three-dimensional, apertured substrate 400 may have one or more arcuate portions, designs, and/or shapes cut out from them to provide an aesthetically pleasing look to the liquid permeable, apertured substrate 400. One side edge 404 may be symmetrical or asymmetrical to another side edge 404 about a longitudinal axis, 408, of the topsheet 24. Likewise, one end edge 406 may be symmetrical or asymmetrical to another side edge 406 about a lateral axis, 410 of the topsheet 24.

The three-dimensional, apertured substrate 400 may comprise one or more layers of nonwoven material. If more than one layer is provided, the layers may be joined together or attached to each other through mechanical bonding, adhesive bonding, pressure bonding, heat bonding, passing heated air through both layers, or by other methods of joining to form the multilayer substrate 400. Alternatively, the layers may be formed in subsequent fiber laydown steps, such as a first and a second carding operation for a first type and a second type of staple fibers or two subsequent beams of spunlaying polymeric filaments comprising additives. The one or more layers may be hydrophobic, with one or more hydrophilic materials proximate to the apertures.

The first layer of the three-dimensional, apertured substrates may comprise a plurality of first fibers and/or filaments (hereafter together referred to as fibers). The plurality of first fibers may comprise fibers that are the same, substantially the same, or different in size, shape, composition, denier, fiber diameter, fiber length, and/or weight. The second layer of the three-dimensional, apertured substrates may comprise a plurality of second fibers. The plurality of second fibers may comprise fibers that are the same, substantially the same, or different in size, shape, composition, denier, fiber diameter, fiber length, and/or weight. The plurality of first fibers may be the same as, substantially the same as, or different than the plurality of second fibers. Additional layers may have the same or different configurations. If a single material is provided as the three-dimensional, apertured substrate, the single layer may have one or more types of fibers. If more than one type of fibers is provided, the fibers may be different in size, shape, composition, denier, fiber dimeter, fiber length and/or weight.

The first layer and/or the second layer, or the single nonwoven substrate, may comprise bicomponent fibers having a sheath and a core. The sheath may comprise polyethylene and the core may comprise polyethylene terephthalate (PET). The sheath and the core may also comprise any other suitable materials known to those of skill in the art. The sheath and the core may each comprise about 50% of the fibers by weight of the fibers, although other variations (e.g., sheath 60%, core 40%; sheath 30%, core 70% etc.) are also within the scope of the present disclosure. The bicomponent fibers or other fibers that make up the first and/or second layers, or the single nonwoven substrate, may have a denier in the range of about 0.5 to about 6, about 0.75 to about 4, about 1.0 to about 4, about 1.2 to about 4, about 1.5 to about 3.5, about 1.5 to about 2.5, about 2, about 3, or about 4, specifically including all 0.1 denier increments within the specified ranges and all ranges formed therein or thereby. Denier is defined as the mass in grams per 9000 meters of a fiber length. In other instances, the denier of the fibers of the first layer may be in the range of about 1 denier to about 4 denier, about 1.2 denier to about 4 denier, 1.5 denier to about 6 denier or about 2 denier to about 4 denier and the denier of the fibers of the second layer may be in the range of about 1 denier to about 4 denier, about 1 denier to about 3.5 denier or about 1 denier to about 3 denier, specifically reciting all 0.1 denier increments within the specified ranges and all ranges formed therein or thereby. In certain instances, the fibers of the first layer may be at least 0.5 denier, at least 1 denier, at least 1.5 denier, or at least 2 denier greater than the denier of the fibers of the second layer depending at least in part on the particular acquisition and/or distribution system in use in a certain absorbent article. In other instances, the denier of the fibers in the first and the second layers may be the same or substantially similar. By providing the fibers of the first layer with a denier higher than a denier of the fibers of the second layer, a pore gradient is provided in the liquid permeable, apertured substrate. This pore gradient may provide better dryness and/or acquisition in the liquid permeable, apertured substrate. The fibers having the larger denier in the first layer provide larger pores than the fibers having the smaller denier in the second layer, thereby producing the pore gradient between the layers.

In some instances, fibers of the first layer may have a lower denier than a denier of the fibers in the second layer. For example, the fibers of the first layer may have a denier of about 2 to about 3 and the fibers of the second layer may have a denier of about 3 to about 4. With both of the layers being hydrophobic, the use of smaller fibers (smaller denier) on the first layer may provide softness, soft glide touch and thicker fibers (larger denier) may be used on the second layer for cushiony softness.

The plurality of first and second fibers (including fibers in the single nonwoven substrate) may also comprise any other suitable types of fibers, such as polypropylene fibers, other polyolefins, other polyesters besides PET such as polylactic acid, thermoplastic starch-containing sustainable resins, other sustainable resins, bio-PE, bio-PP, and Bio-PET, viscose fibers, rayon fibers, or other suitable nonwoven fibers, for example. These fibers may have any suitable deniers or denier ranges and/or fiber lengths or fiber length ranges. The plurality of first and second fibers (including the fibers in the single nonwoven substrate) may remain in their natural hydrophobic state or may be further treated to become more hydrophobic.

The first layer may have a basis weight in the range of about 10 gsm to about 25 gsm. The second layer may have a basis weight in the range of about 10 gsm to about 45 gsm. The basis weight of the substrate (both first and second layers, or the single layer version) may be in the range of about 20 gsm to about 70 gsm, about 20 gsm to about 60 gsm, about 25 gsm to about 50 gsm, about 30 gsm to about 40 gsm, about 30gsm, about 35 gsm, or about 40 gsm, for example.

In a form, the basis weight of the substrate may be about 30 gsm to about 40 gsm or about 35 gsm. In an example, the first layer may have a basis weight in the range of about 10 gsm to about 20 gsm, or about 15 gsm, and the second layer may have a basis weight in the range of about 15 gsm to about 25 gsm, or about 20 gsm. In another example, the basis weight of the substrate may be about 20 gsm. In such an example, the first layer may have a basis weight of about 10 gsm and the second layer may have a basis weight of about 10 gsm. In still another example, the basis weight of the substrate may be about 60 gsm. In such an example, the first layer may have a basis weight of about 24 gsm, and the second layer may have a basis weight of 36 gsm. All other suitable basis weight ranges for the first and second layers of the substrates, or the single layer substrates, are within the scope of the present disclosure. Accordingly, the basis weight of the layers of the substrates, or the single layer substrates, may be designed for specific product requirements.

Specifically recited herein are all 0.1 gsm increments within the above-specified ranges of basis weight and all ranges formed therein or thereby. All basis weight numbers of the present disclosure are measured according to the Basis Weight Test herein.

In some instances, it may be desirable to have a higher basis weight in the first layer compared to the second layer. For instance, the first layer's basis weight may be at least about 1 to about 4 times, at least about 1 to about 3.5 times, about 1.5 to about 3 times, about 1.5 times to about 3 times, about 2 times, about 2.5 times, or about 3 times greater than the second layer's basis weight. In some instances, the basis weight of the first layer may be in the range of about 20 gsm to about 30 gsm, and the basis weight of the second layer may be in the range of about 10 gsm to about 20 gsm, for example. Specifically recited herein are all 0.1 gsm increments within the above-specified ranges of basis weight and all ranges formed therein or thereby.

In some instances, it may be desirable to have a higher basis weight in the second layer compared to the first layer. For instance, the second layer's basis weight may be at least about 1 to about 4 times, at least about 1 to about 3.5 times, about 1.5 to about 3 times, about 1.5 times to about 3 times, about 2 times, about 2.5 times, or about 3 times greater than the first layer's basis weight. In some instances, the basis weight of the second layer may be in the range of about 20 gsm to about 30 gsm, and the basis weight of the first layer may be in the range of about 10 gsm to about 20 gsm, for example. Specifically recited herein are all 0.1 gsm increments within the above-specified ranges of basis weight and all ranges formed therein or thereby.

In still other instances, the basis weight of the first and second layer may be the same, or substantially the same.

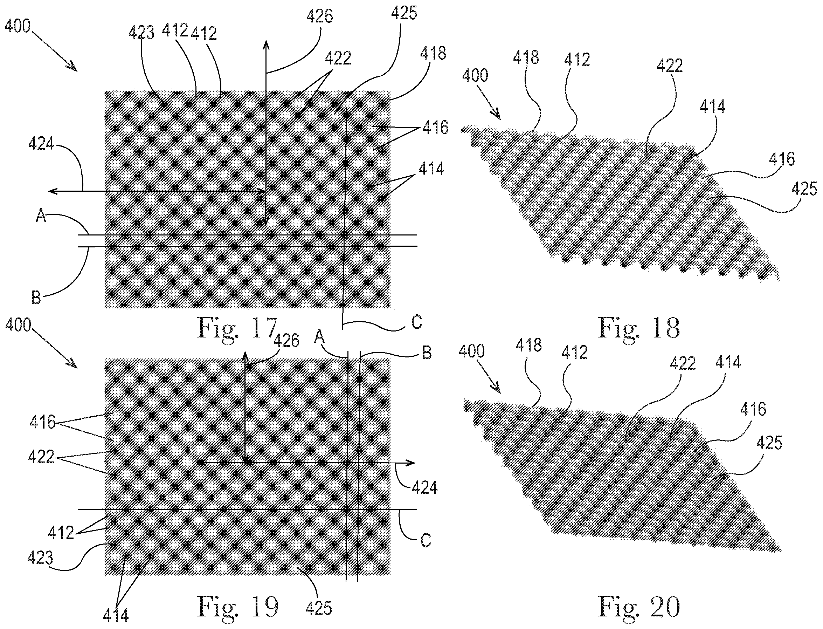

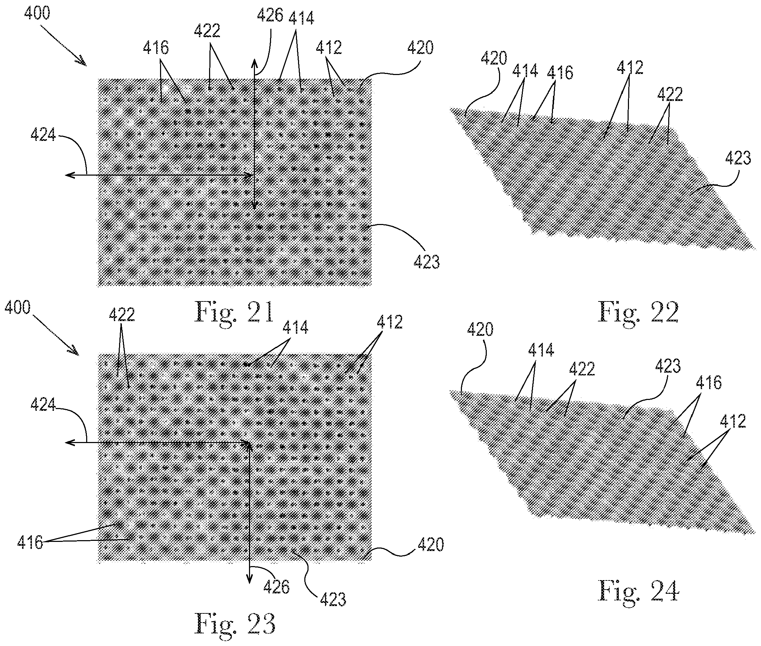

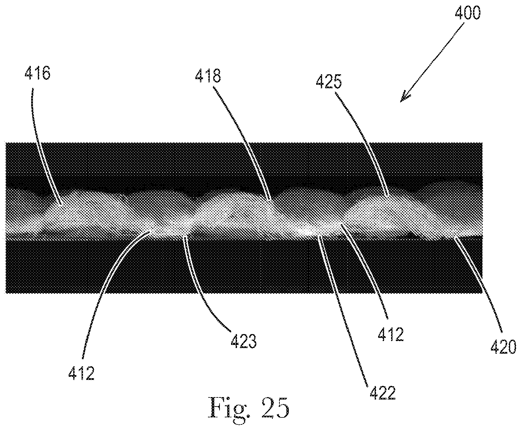

Fig. 17 is a front view of a portion of a three-dimensional, apertured, nonwoven, liquid permeable substrate, wearer-facing surface facing the viewer. Fig. 18 is a front perspective view of the portion of the three-dimensional, apertured, nonwoven, liquid permeable substrate of Fig. 17. Fig. 19 is another front view of a portion of a three-dimensional, apertured, nonwoven, liquid permeable substrate, wearer-facing surface facing the viewer. Fig. 20 is a front perspective view of the portion of the substrate of Fig. 19. Fig. 21 is a back view of a portion of a three-dimensional, apertured, nonwoven, liquid permeable substrate, wearer-facing surface facing the viewer. Fig. 22 is a back perspective view of the portion of the substrate of Fig. 21. Fig. 23 is another back view of a portion of a three-dimensional, apertured, nonwoven, liquid permeable substrate, wearer-facing surface facing the viewer. Fig. 24 is a back perspective view of the portion of the substrate of Fig. 23. Fig. 25 is a cross-sectional view of a three-dimensional, apertured, nonwoven, liquid permeable substrate of the present disclosure.

Referring generally to Figs. 17-25, the liquid permeable substrate 400 may comprise a first layer and a second layer, a single layer, or more than two layers. The substrate 400 may comprise a plurality of land areas 412, a plurality of recesses 414, and a plurality of projections 416. The plurality of projections 416 may form the first elements having the first z-directional height, and the land areas 412 may form the second elements having the second z-direction height, as described above. The plurality of land areas 412, the plurality of recesses 414, and the plurality of projections 416 may together form a first three-dimensional surface on a first side 418 of the substrate 400. The plurality of land areas 412, the plurality of recesses 414, and the plurality of projections 416 may also form a second three-dimensional surface on a second side 420 of the substrate 400. The projections 416 may be generally dome shaped on a wearer-facing surface of the liquid permeable substrate 400 and may be hollow arch-shaped on the garment-facing surface of the substrate 400. All of, or a majority of (i.e., more than 50% of, or more than 75% of), or substantially all of, the recesses 414 may define an aperture 422 therein at a location most distal from a top peak 425 of an adjacent projection 416. A perimeter 423 of a majority of, or all of, the apertures 422 may form a bottommost portion or plane of the substrate 400, while the top peak 425 (i.e., uppermost portion) of a majority of, or all of, the projections 416 may form a topmost portion or plane of the substrate 400. The apertures 422 may extend through the first and the second layers of the substrate 400.

The land areas 412 may be positioned intermediate: (1) adjacent projections 416, (2) adjacent recesses 414 and/or adjacent apertures 422. The land areas 412 may also surround at least a portion of, or all of, a majority of, or all of, the recesses 414 and/or the apertures and at least a majority of, or all of, the projections 416. The land areas 412 may be positioned between a plane of a perimeter of at least a majority of the apertures 422 and a plane of at least a majority of the top peaks 425 of the projections 416.

The projections 416 may alternate with the recesses 414 and/or the apertures 422 in a direction generally parallel with a lateral axis 424 of the liquid permeable substrate 400. The lateral axis 424 is generally parallel with the lateral axis 410 illustrated in Figs. 14-16. The projections 416 may also alternate with the recesses 414 and/or apertures 422 in a direction generally parallel with a longitudinal axis 426 of the liquid permeable substrate 400. The longitudinal axis 426 is generally parallel with the longitudinal axis 408 illustrated in Figs. 14-16. In such a configuration, in a direction generally parallel with the lateral axis 424 or in a direction generally parallel with the longitudinal axis 426, the projections 416 and the recesses 414 and/or apertures 422 alternate (i.e., projection, recess and/or apertures, projection, recess and/or aperture). This feature provides better softness to the substrate 400 in that there is a soft projection peak 425 intermediate most of, or all of, adjacent recesses 414 and/or apertures 422. This feature also helps maintain the skin of a wearer away from fluids in the land areas 412 and/or the recesses 414, since the projections 416 essentially create a spacer between the skin and the fluids.

Two or more adjacent projections 416 may be separated from each other by a recess 414 and/or an aperture 422 and one or more land areas 412 in a direction generally parallel to the lateral axis 424 or in a direction generally parallel to the longitudinal axis 426. Two or more adjacent recesses 414 and/or apertures 422 may be separated by a projection 416 and one or more land areas 412 in a direction generally parallel to the lateral axis 424 or in a direction generally parallel to the longitudinal axis 426. The land areas 412 may fully surround the apertures 422 and the projections 416. The land areas 412 may together form a generally continuous grid through the substrate 400, while the projections 416 and the recesses 414 and/or the apertures 422 may be discrete elements throughout the substrate.

In some instances, two or more, such as four projections 416 may be positioned around at least a majority of, substantially all of, or all of, the recesses 414 and/or the apertures 422 (this does not include the land areas 412 intermediate the projections 416 and the recesses 414 and/or the apertures 422). Two or more recesses 414 and/or apertures 422, such as four, may be positioned around at least a majority of, substantially all of, or all of, the projections 416 (this does not include the land areas 412 intermediate the recesses 414 and/or the apertures 422 and the projections 416). The projections 416, recesses 414, apertures 422, and land areas 412 may all be formed of portions of the first and second layers of the substrate (or in the single layer if only a single layer is provided). If more than two layers are provided in a substrate, the projections 416, recesses 414, apertures 422, and land areas 412 may all be formed of portions of the first, second and third layers of the substrate. The same may be true if more than three layers are provided in a particular substrate. In other instances, the land areas 412 may only be formed in the first layer.

The apertures 422 and/or the recesses 414 may comprise a first set of apertures and/or recesses 414 together forming a first line in the substrate 400 and a second set of apertures 422 and/or recesses 414 together forming a second line in the substrate 400. The first line may be generally parallel with or generally perpendicular to the second line. The first line may also form an acute or obtuse angle with the second line. The projections 416 may comprise a first set of projections 416 together forming a first line in the substrate 400 and a second set of projections 416 together forming a second line in the substrate 400. The first line may be generally parallel with or generally perpendicular to the second line. The first line may also form an acute or obtuse angle with the second line.

The substrate 400 may be generally symmetrical about the lateral axis 424 and/or generally symmetrical about the longitudinal axis 426. In other instances, the substrate may not be symmetrical about the lateral axis 424 and/or the longitudinal axis 426.

In one form, the substrate 400 may comprise a first line comprising alternating apertures 422 and projections 416 extending in a direction parallel to the lateral axis 424 and a second adjacent line comprising alternating apertures 422 and projections 416 extending in the direction generally parallel to the lateral axis 424. The lines will run through the center of the apertures 422 and the projections 416. See for, example, Fig. 17, lines A and B. If a line, C, is drawn in a direction generally parallel to the longitudinal axis 426 and that intersects lines A and B, an aperture 422 will be located at the intersection of lines A and C and a projection 416 will be located at the intersection of the lines B and C. The same is true if lines A and B are drawn in a direction parallel to the longitudinal axis 426 and line C is draw in a direction generally parallel to the lateral axis 424, as illustrated in Fig. 19. If the lines are drawn at different locations, the intersection of lines A and C may have a projection 416 and the intersection of lines B and C may have an aperture 422. The main point being that the rows of apertures and the rows of projections are staggered. By staggering the apertures and projections in this fashion, better softness is achieved in the wearer-facing surface of the substrate 400 owing to a soft projection or projection crest being intermediate two apertures.

### Parameters of the Three-Dimensional Substrates

All or a majority of the projections 416 may have a z-directional height in the range of about 300 µm to about 6000 µm, about 400 µm to about 5000 µm, about 400 µm to about 4000 µm, about 300 µm to about 3000 µm, about 500 µm to about 3000 µm, about 500 µm to about 2000 µm, about 750 µm to about 1500 µm, about 800 µm to about 1400 µm, about 900 µm to about 1300 µm, about 1000 µm to about 1300 µm, about 1100 µm to about 1200 µm, about 1165, about 1166, about 1167, or about 1150 µm to about 1200 µm, specifically reciting all 1 µm increments within the above-specified ranges and all ranges formed therein or thereby. The z-directional height of the projections 416 are measured according to the Projection Height Test described herein.

All or a majority of the recesses 414 may have a z-directional height in the range of about 200 µm to about 3000 µm, about 300 µm to about 2000 µm, about 100 µm to about 2000 µm, about 400 µm to about 2000 µm, about 500 µm to about 1500 µm, about 700 µm to about 1300 µm, about 800 µm to about 1200 µm, about 900 µm to about 1100 µm, about 900 µm to about 1000 µm, about 970 µm, or about 950 µm to about 1000 µm, specifically reciting all 1 µm increments within the above-specified ranges and all ranges formed therein or thereby. The z-directional height of the recesses 416 are measured according to the Recess Height Test described herein.

The substrate, 400, or portions thereof, may have an overall z-directional height in the range of about 500 µm to about 6000 µm, about 800 µm to about 6000 µm, about 750 µm to about 4000 µm, about 1000 µm to about 6000 µm, about 1500 µm to about 6000 µm, about 1000 µm to about 3000 µm, about 1500 µm to about 2500 µm, about 1750 µm to about 2300 µm, about 1900 µm to about 2300 µm, about 2000 µm to about 2300 µm, about 2100 µm to about 2250 µm, about 2136 µm, or about 2135 µm, specifically reciting all 1 µm increments within the above-specified ranges and all ranges formed therein or thereby. The overall z-directional height of the substrate 400, or portions thereof, is measured according to the Overall Substrate Height Test described herein.

A majority of, or all of, the apertures 422 may have an effective aperture area in the range of about 0.4mm² to about 10mm², about 0.5mm² to about 8mm², about 0.5 mm² to about 3 mm², about 0.5 mm² to about 4 mm², about 0.5 mm² to about 5 mm², about 0.7mm² to about 6mm², about 0.7mm² to about 3mm², about 1 mm² to about 4 mm², about 1 mm² to about 5 mm², about 0.8mm² to about 3mm², about 0.9mm² to about 1.4mm², about 1mm², about 1.1mm², about 1.2mm², about 1.23mm², about 1.3mm², or about 1.4mm², specifically reciting all 0.1 mm² increments within the above-specified ranges and all ranges formed therein or thereby. The effective aperture area of the apertures is measured according to the Aperture Test described herein.

A majority of, or all of, the apertures 422 may have a feret (length of aperture) in the range of about 0.5mm to about 4mm, about 0.8mm to about 3mm, about 1mm to about 2mm, about 1.2mm to about 1.8mm, about 1.4mm to about 1.6mm, about 1.49, or about 1.5mm specifically reciting all 0.1 mm increments within the above-specified ranges and all ranges formed therein or thereby. The aperture feret is measured according to the Aperture Test described herein.

A majority of, or all of, the apertures 422 may have a minimum feret (width of aperture) in the range of about 0.5mm to about 4mm, about 0.7mm to about 3mm, about 0.8mm to about 2mm, about 0.9mm to about 1.3mm, about 1mm to about 1.2mm, about 1mm, about 1.1mm, about 1.11mm, about 1.2mm, or about 1.3mm, specifically reciting all 0.1mm increments within the above-specified ranges and all ranges formed therein or thereby. The aperture minimum feret is measured according to the Aperture Test described herein.

A majority of, or all of, the apertures 422 may have a feret to minimum feret ratio in the range of about 0.3 to about 2.5, about 0.5 to about 2, about 0.8 to about 1.6, about 1 to about 1.5, about 1.1 to about 1.5, about 1.2, about 1.3, about 1.35, about 1.4, or about 1.5, specifically reciting all 0.1 increments within the above-specified ranges and all ranges formed therein or thereby. The feret ratio is calculated by dividing the aperture feret by the aperture minimum feret.

The average lateral axis center-to-center aperture spacing of a majority of, or all of, adjacent apertures, measuring across a projection, is in the range of about 2mm to about 20mm, about 2mm to about 15mm, about 3mm to about 12mm, about 3mm to about 10mm, about 3mm to about 8mm, about 3mm to about 7mm, about 4mm, about 5mm, about 6mm, about 7mm, about 4mm to about 6mm, about 5mm to about 6mm, about 4.8mm, about 4.9mm, about 5.0mm, about 5.1mm, about 5.2mm, about 5.3mm, about 5.4mm, about 5.5mm, about 5.6mm, about 5.7mm, about 5.8mm, or about 5.9mm, specifically reciting all 0.1 mm increments within the above-specified ranges and all ranges formed therein or thereby. The average lateral axis center-to-center spacing of adjacent apertures is measured according to the Average Aperture Spacing Test (Lateral Axis Aperture Spacing) described herein.

The average longitudinal axis center-to-center aperture spacing of a majority of, or all of, adjacent apertures, measuring across a projection, is in the range of about 2mm to about 20mm, about 2mm to about 15mm, about 3mm to about 12mm, about 3mm to about 10mm, about 3mm to about 8mm, about 3mm to about 7mm, about 4mm, about 5mm, about 6mm, about 7mm, about 4mm to about 6mm, about 5mm to about 6mm, about 4.8mm, about 4.9mm, about 5.0mm, about 5.1mm, about 5.2mm, about 5.3mm, about 5.4mm, about 5.5mm, about 5.6mm, about 5.7mm, about 5.8mm, or about 5.9mm, specifically reciting all 0.1 mm increments within the above-specified ranges and all ranges formed therein or thereby. The average longitudinal axis center-to-center spacing of adjacent apertures is measured according to the Average Aperture Spacing Test (Longitudinal Axis Aperture Spacing) described herein.

A majority of, or all of, the projections 416 may have a widest cross-sectional diameter, taken in a direction parallel to the lateral axis of the absorbent article, in the range of about 1,, to about 15mm, about 1mm to about 10mm, about 1mm to about 8mm, about 1mm to about 6mm, about 1.5mm to about 6mm, about 2mm to about 5mm, specifically reciting all 0.1 mm increments within the above-specified ranges and all ranges formed therein or thereby.

A majority of, or all of, the projections 416 may have a widest cross-sectional diameter, taken in a direction parallel to the longitudinal axis of the absorbent article, in the range of about 1mm to about 15mm, about 1mm to about 10mm, about 1mm to about 8mm, about 1mm to about 6mm, about 1.5mm to about 6mm, about 2mm to about 5mm, specifically reciting all 0.1 mm increments within the above-specified ranges and all ranges formed therein or thereby.

The substrates of the present disclosure may have a % effective open area in the range of about 1% to about 50%, about 1% to about 40%, about 3% to about 35%, about 5% to about 25%, about 5% to about 20%, about 6% to about 18%, about 5% to about 15%, about 9% to about 13%, about 8%, about 9%, about 10%, about 11%, about 12%, about 13%, or about 14%, specifically reciting all 0.1% increments within the above-specified ranges and all ranges formed therein or thereby. The % effective open area of the substrates is measured according to the Aperture Test described herein.

The substrates of the present disclosure may have apertures having a perimeter in the range of about 1mm to about 50mm, about 1mm to about 30mm, about 2mm to about 20mm, about 2mm to about 15mm, about 2mm to about 10mm, about 3mm to about 8mm, about 4mm, about 5mm, about 5.42mm, about 6mm, or about 7mm, specifically reciting all 0.1mm increments within the above-specified ranges and all ranges formed therein or thereby. The perimeter of the apertures is measured according to the Aperture Test described herein.

### Ratios

The ratio of the height of the projections (µm) to the % effective open area may be in the range of about 70 to about 160, about 80 to about 150, about 100 to about 145, about 95 to about 150, about 100 to about 140, about 110 to about 130, about 115 to about 130, about 118 to about 125, about 120, about 121, about 122, about 122.74, about 123, or about 124, specifically reciting all 0.1 increments within the specified ranges and all ranges formed therein or thereby.

The ratio of the overall substrate height (µm) to the % effective open area may be in the range of about 125 to about 350, about 150 to about 300, about 175 to about 275, about 200 to about 250, about 215 to about 235, about 220 to about 230, or about 225, specifically reciting all 0.1 increments within the specified ranges and all ranges formed therein or thereby.

The substrates of the present disclosure may comprise one or more colors, dyes, inks, indicias, patterns, embossments, and/or graphics. The colors, dyes, inks, indicias, patterns, and/or graphics may aid the aesthetic appearance of the substrates.

The substrates of the present disclosure may be used as a portion of, or all of, any suitable products, such as dusters, wipes (wet or dry), absorbent articles, makeup removal substrates, paper towels, toilet tissue, facial tissue, medical gowns, surgical substrates, wraps, filtration substrates, or any other suitable products.

### Method of Making the Three-Dimensional, Apertured, Substrates or Absorbent Articles Comprising the Three-Dimensional, Apertured Substrates

The three-dimensional, apertured, nonwoven substrates and absorbent articles comprising three-dimensional, apertured, nonwoven substrates of the present disclosure may be made by hand or industrially produced at high speed.

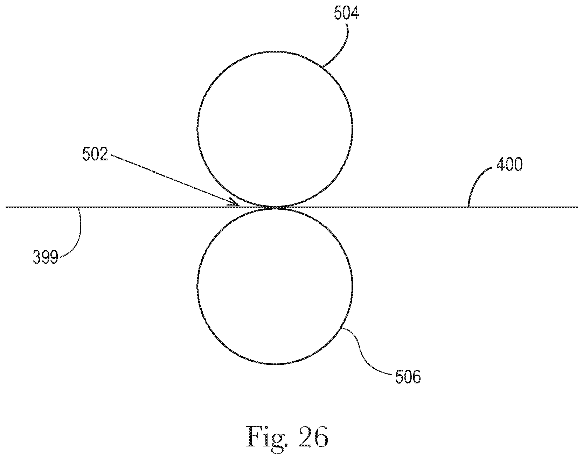

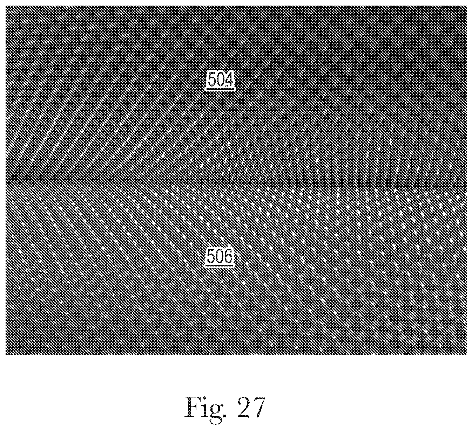

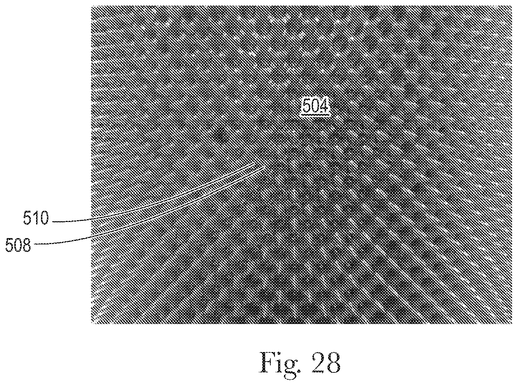

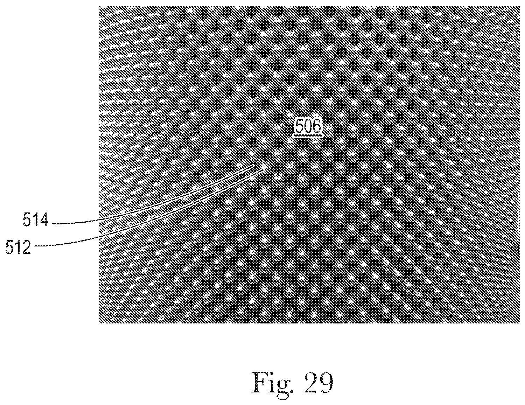

Fig. 26 is a schematic illustration of one example process for forming the substrates of the present disclosure. Fig. 27 is a view of intermeshing engagement of portions of first and second rolls. Fig. 28 is a view of a portion of the first roll. Fig. 29 is a view of a portion of the second roll.

Referring to Figs. 26-29, the substrates of the present disclosure may be formed by passing a one or more layer substrate 399 (non-three dimensional) through a nip 502 formed by two intermeshing rolls 504 and 506 to form a three-dimensional substrate 400. The rolls 504 and 506 may be heated. A first roll 504 may create the apertures 422 and the recesses 414 in the substrate 400 (in combination with the second roll) and a second roll 506 may create the projections 416 in the substrate 400 (in combination with the first roll). The first roll 504 may comprise a plurality of conically-shaped protrusions 508 extending radially outwardly from the first roll 504. The first roll 504 may also comprise a plurality of recesses 510 formed in a radial outer surface of the first roll 504. The second roll 506 may comprise a plurality of dome-shaped protrusions 512 extending radially outwardly from the second roll 506. The second roll 506 may also comprise a plurality of recesses 514 formed in the radial outer surface of the second roll 506. The protrusions 508 on the first roll 504 may have a different size, shape, height, area, width and/or dimension than the protrusions 512 on the second roll 506. The recesses 510 formed in the first roll 504 may have a different size, shape, height, area, width, and/or dimension than the recesses 514 formed in the second roll 506. The recesses 510 in the first roll 504 may be configured to at least partially receive the dome-shaped protrusions 512, thereby creating the projections 414 in the substrate 400. The recesses 510 may be deep enough so that the portions of the substrate forming the projections 414 and projection peaks 425 will not be compressed, or sufficiently compressed. Specifically, as the dome-shaped protrusions 512 engage into the recesses 510, there is sufficient depth left in the space between the surfaces in a radial direction so that the thickness of the substrate in the projections 414 is higher than the thickness of the recesses 510. This feature provides projections 414 with a softer feel and a greater height compared to compressing the portions of the substrate forming the projections. The recesses 514 in the second roll 506 may be configured to at least partially receive the conically-shaped protrusions 508 thereby creating the recesses 414 and the apertures 422 in the substrate 400.

### Packages

The absorbent articles of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 110 mm, less than about 105 mm, less than about 100 mm, less than about 95 mm, less than about 90 mm, less than about 85 mm, less than about 80 mm, less than about 78 mm, less than about 76 mm, less than about 74 mm, less than about 72mm, or less than about 70 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 110 mm, from about 70 mm to about 105 mm, from about 70 mm to about 100 mm, from about 70 mm to about 95 mm, from about 70 mm to about 90 mm, from about 70 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

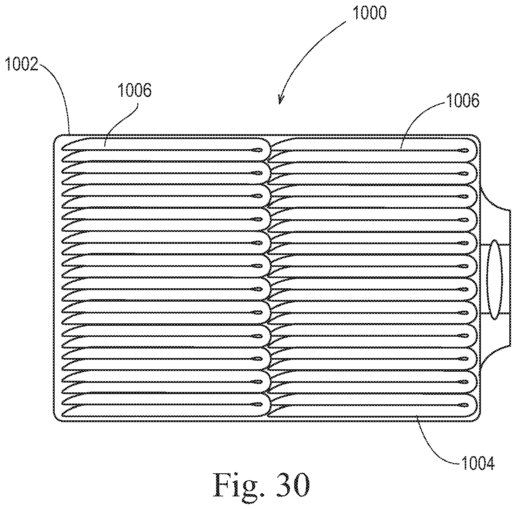

Fig. 30 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

As mentioned above, these three-dimensional apertured, nonwoven substrates 400 (e.g., used as topsheets) may be hydrophobic materials, such as hydrophobic nonwoven materials, but hydrophilic materials may be provided only around perimeters 423 of the apertures 422 and/or only in areas closely surrounding the apertures 422, such as within about 1mm of the perimeters of the apertures 422 or extending into portions of the recesses 414. The hydrophilic materials in areas closely surrounding the apertures 422 may be in the recesses 414. The hydrophilic materials may also be present in walls (thicknesses of the perimeters) of the apertures. By providing three-dimensional apertured, hydrophobic substrates, with hydrophilic materials proximate to the apertures, fluids may be quickly wicked through the apertures, even with otherwise hydrophobic substrates. Owing to the addition of the hydrophilic material proximate to the apertures, the apertures now may be reduced in size and/or area compared to conventional apertured hydrophobic topsheets, thereby reducing skin marking and rewet, while achieving about the same run-off and leakage benefits as the fully hydrophobic topsheets with the larger apertures. Upon information and belief, the addition of the hydrophilic materials of the present disclosure on the areas proximate to the apertures, or at least around the aperture perimeters, may reduce the breakthrough pressure which is required for the fluids to be imbibed into the substrate.

Thus, the three-dimensional, apertured substrates or topsheets of the present disclosure solve the problems of both fully hydrophilic topsheets and fully hydrophobic topsheets, leave the wearer's skin dry, reduce fluid/skin contact, allow for smaller apertures, reduced skin marking and rewet, and reduce run-off and leakage problems.

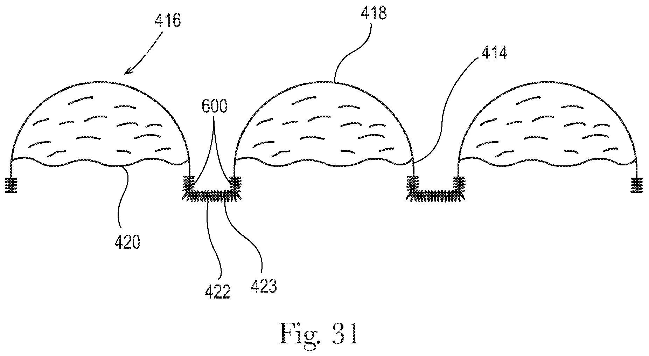

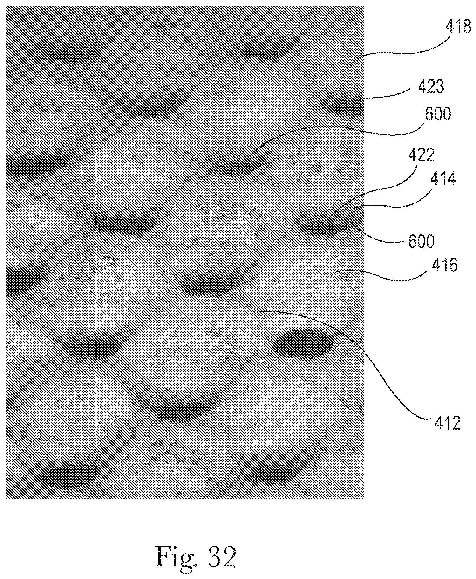

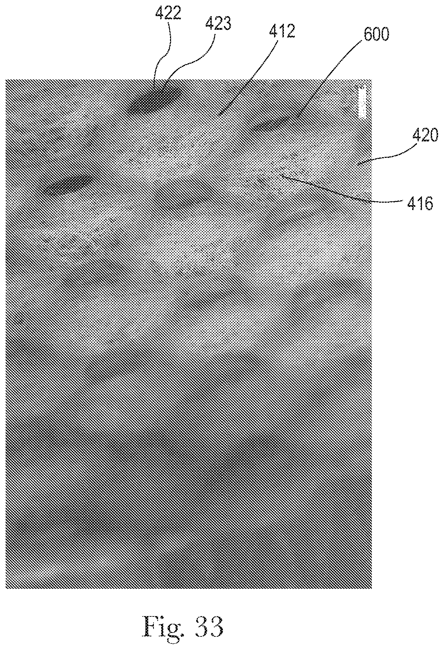

Fig. 31 is a schematic illustration of a cross-sectional view of an example of a portion of a three-dimensional, apertured nonwoven substrates of the present disclosure for purposes of illustration. Fig. 32 is a photograph of an example three-dimensional, apertured nonwoven substrate of the present disclosure, wearer-facing surface facing upwards. Fig. 33 is a photograph of an example three-dimensional, apertured nonwoven substrate of the present disclosure, garment-facing surface facing upwards. Referring to Figs. 31-33, the three-dimensional, apertured nonwoven substrates 400 comprise one or more hydrophobic layers, but with hydrophilic material applied proximate to the apertures 422. The hydrophilic material may be positioned in walls of the apertures 422, on portions of the recesses 414, or in materials proximate to the apertures. The projections 416 and the land areas 412 may typically be free of any of the hydrophobic material. As a result, when fluids contact the three-dimensional, apertured nonwoven substrates (e.g., topsheets), the fluids primarily do not penetrate the land areas 412 and the projections 416, but instead are driven by gravity towards the recesses 414. Once the fluids arrive at the recesses 414, they are quickly wicked through the apertures 422 owing to the hydrophilic materials 600 positioned proximate to the apertures 422 and/or about the perimeter 423 of the apertures 422. Without the hydrophilic materials 600, the fluids would sit within the recesses 414 and not be wicked through the apertures 422 due to the small effective apertures areas (e.g., creating capillary forces that resist movement of the fluids through the apertures). Example "small" effective aperture areas may be in the range of from about 1 mm² to about 4.0 mm², about 1 mm² to about 3.5 mm², of about 1 mm² to about 3 mm², of about 1.2 mm² to about 2.5 mm², or of about 1.5 mm² to about 2.5 mm², specifically reciting all 0.1 mm² increments within the specified ranges and all ranges formed therein or thereby. Effective aperture area is measured according to the Aperture Test herein. If the apertures have a conical shape, effective aperture area is taken at the minimum dimension of the conical shape.

If the recesses 414 had vertical walls or even if areas at the top of the recesses had smaller areas compared to the apertures 422, fluids will still be able to be wicked through the apertures owing to the hydrophilic material 600. The hydrophilic materials allow for different recess configurations compared to fully hydrophobic substrates. In fully hydrophobic substrates, the recesses would have to be upside down conical shapes and the apertures would have to be quite large.

Furthermore, by providing the hydrophilic material 600 proximate to the apertures, the apertures walls (i.e., thickness of the aperture perimeter) may be about 0.3mm thick, about 0.6mm thick, or about 1 mm thick, for example, and may still wick fluids therethrough, even with the smaller effective aperture areas. This would not be possible with a fully hydrophobic substrate with the same effective aperture areas.

Some suitable examples of hydrophilic materials comprise a surfactant or combination of surfactants with hydrophilic/lyophilic balance number (HLB) of greater than or equal to about 7, greater than or equal to about 10, or greater than or equal to about 14. Hydrophilic materials that do not generally have a measured HLB may also be used.

Some suitable examples of hydrophilic materials include non-ionic surfactants including esters, amides, carboxylic acids, alcohols, ethers - polyoxyethylene, polyoxypropylene, sorbitan, ethoxylated fatty alcohols, alyl phenol polyethoxylates, lecithin, glycerol esters and their ethoxylates, and sugar based surfactants (polysorbates, polyglycosides). Other suitable nonionic surfactants include: ethoxylates, including fatty acid ester ethoxylates, fatty acid ether ethoxylates, and ethoxylated sugar derivatives (e.g., ethoxylated fatty acid polyesters, ethoxylated fatty acid sorbitan esters, and the like), and the like, as well as combinations comprising at least one of the foregoing. Other suitable examples include anionic surfactants including sulfonates, sulfates, phosphates, alkali metal salts of fatty acids, fatty alcohol monoesters of sulfuric acid, linear alkyl benzene sulfonates, alkyl diphenyloxide sulfonates, lignin sulfonates, olefin sulfonates, sulfosuccinates, and sulfated ethoxylates of fatty alcohols. Other suitable examples include cationic surfactants including amines (primary, secondary, tertiary), quaternary ammoniums, pyridinium, quaternary ammonium salts- QUATS, alkylated pyridinium salts, alkyl primary, secondary, tertiary amines, and alkanolamides. Other suitable examples include zwiterionic surfactants including amino acids and derivatives, amine oxide, betaines, and alkyl amine oxides. Other suitable examples include polymeric surfactants including polyamines, carboxylic acid polymers and copolymers, EO/PO block copolymers, ethylene oxide polymers and copolymers, and polyvinylpyrrolidone. Other suitable examples include silicone surfactants including dimethyl siloxane polymers with hydrophile. And other suitable examples include perfluorocarboxylic acid salts and fluorosurfactants.

The hydrophilic materials that do not generally have a measured HLB may also be used. Such hydrophilic materials may include, without limitation, diols, such as glycols and polyglycols. Suitable nonionic surfactants include, but are not intended to be limited to, C2-8 diols and polyglycols, and the like. Generally, the diol may be glycols (C2 and C3 diols) and polyglycols. The term "polyglycol" refers to a dihydroxy ether formed by dehydration of two or more glycol molecules. A representative, non-limiting list of useful polyglycols, includes: ethylene glycol, propylene glycol, polyethylene glycols, polypropylene glycols, methoxypolyethylene glycols, polybutylene glycols, block copolymers of butylene oxide and ethylene oxide, and the like, as well as combinations comprising at least one of the foregoing.

Additionally, suitable hydrophilic materials include finishing treatments which are typically proprietary blends of synthetic surfactant solutions which are commercially available. Examples include materials from Schill & Seilacher AG under the tradename Silastol (e.g. Silastol PHP 26, Silastol PHP 90, Silastol PST-N, Silastol PHP 207, Silastol PHP 28 & Silastol PHP 8), from Pulcra Chemicals under the tradename Stantex^{®} (e.g., Stantex S 6087-4, & Stantex PP 602), among others.

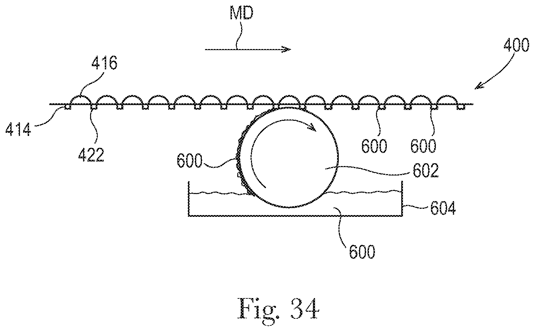

The hydrophilic materials may be applied to the substrate using the printing methods described in U.S. Patent Application Serial No. 62/385,265, P&G Attorney Docket No. 14495PQ, filed on September 9, 2016. The hydrophilic materials may also be applied by the process illustrated in Fig. 34. In Fig. 34, a roller 602 is positioned between the three-dimensional, apertured substrate 400 and a vat 604 containing liquid hydrophilic material 600. The substrate 400 is being conveyed in the direction of arrow MD. The substrate 400 is shown in cross-section for illustration purposes. The roller 602 may be rotating in the direction shown by the arrow on the roller 602, or may rotate in the opposite direction. The roller 602 is configured to pick up the liquid hydrophilic material 600 from the vat 604 and carry a film of the liquid hydrophilic material 600 on its outer surface and then deposit some of the liquid hydrophilic material 600 on portions of the recesses 414 and/or proximate to the apertures 422. The roller 602 may be positioned such that it only engages the recesses 414 and/or areas proximate to the apertures 422. The perimeters 423 of the apertures 422 generally form the lowest plane of the substrates 400. Thus, the roller 602 primarily contacts the perimeters 423 of the apertures 422 and some of the hydrophilic material wicks to areas proximate to the recesses 414. In such a fashion, the hydrophilic material may be applied to the recesses and/or areas proximate to the apertures, but not to the hydrophobic land areas 412 and projections 416. The outer surface of the roller 602 may match the speed of the moving substrate or may be run is faster or slower than the moving substrate. The tension of the substrate 400 and wrap angle may need to be adjusted to limit the contact of the substrate 400 to only areas proximate to the apertures 422. The pressure the roller 602 applies to the substrate 400 in the nip, N, may also be adjusted to apply less or more of the hydrophobic materials to the substrates 400.

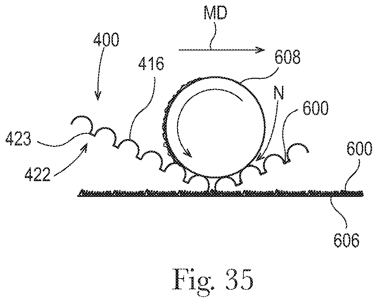

The hydrophilic materials may also be applied proximate to apertures 422 of the substrates 400 by the process illustrated in Fig. 35. Referring to Fig. 35, a hydrophilic material 600 (e.g., 1.5% S6327 solution) is spread on a flat, clean surface 606. The substrate 400 is conveyed through a nip, N, created between the surface 606 and a roller 608. The substrate 400 may be generally moving in the direction indicated by the arrow, MD. The roller 608 may be rotating in the direction indicated by the arrow on the roller 608. The roller 608 may be present to ensure contact between the hydrophobic material 600 and the areas of the substrate proximate to the apertures 422. The tension of the substrate 400 and wrap angle may need to be adjusted to limit the contact of the substrate 400 to only areas proximate to the apertures 422. The pressure the roller 608 applies to the substrate 400 in the nip, N, may also be adjusted to apply more or less of the hydrophobic materials to the substrates 400.

### Examples:

A single variable experiment was run using three different three-dimensional, apertured, nonwoven substrates. The ***first three-dimensional, apertured nonwoven substrate*** was fully hydrophobic and did not have any of the hydrophilic materials applied thereto. The ***second three-dimensional, apertured substrate*** had the hydrophilic material applied proximate to the apertures and portion of the recesses as described herein. The second three-dimensional, apertured substrate was obtained by selectively depositing the hydrophobic material as illustrated in Fig. 35. Use of a blue pigment and Raman spectroscopy were used to confirm that the hydrophilic materials had been indeed selectively applied proximate to the apertures as intended. The ***third three-dimensional, apertured substrate*** had a wearer-facing surface hydrophobic layer and a garment-facing surface hydrophilic layer. Other than the hydrophobic/hydrophilic differences the first, second, and third three-dimensional apertured substrates were the same.

Diaper prototypes were created using Pampers^{®} Swaddlers^{®} size 2 diapers commercially available in the United States in October of 2014. Pampers^{®} Swaddlers size 2 diapers each comprise a topsheet, an acquisition layer beneath the topsheet, a distribution layer beneath the acquisition layer, an absorbent core between the distribution layer and a backsheet beneath the absorbent core. The commercial topsheets of the Pampers^{®} Swaddlers diapers were replaced with the first substrate, the second substrate, and the third substrate by the process described below. For each diaper prototype a 5 gsm spiral adhesive was applied on the acquisition layer and the first, second, and third substrates were placed across the full length/width with the aperture perimeters facing the absorbent core. Each prototype diaper was stored for 3 days at 23 °C +/- 2 °C, and 50% +/- 10% Relative Humidity (RH) before testing.

The first, second, and third three-dimensional, apertured substrates were each applied to the Swaddlers^{®} Size 2 diaper, as the topsheet, as follows. First, the barrier leg cuff tackdown bonds were opened. Next, the topsheets of the Swaddlers^{®} products were removed using freeze spray. Then, the first, second, and third three-dimensional substrates having the same size (i.e., length and width) as the removed topsheet where applied to the Swaddlers^{®} products. The first, second, and third three-dimensional substrates were attached to the acquisition layer of the Swaddlers^{®} products using the 5mg spiral glue. The leg cuff tackdown bonds were then re-created using double sided tape.

The prototypes so produced were tested in a lab for topsheet run-off, according to the Run-Off Test herein. Below are the results.

### Run-Off

**Table 1**

| The reported data is the average and the standard deviation is in parenthesis. | | | |
|---|---|---|---|
| | First Substrate on Swaddlers^{®} | Second Substrate on Swaddlers^{®} (*Present Disclosure*) | Third Substrate on Swaddlers^{®} |
| TS Run-off Gush 1, g | 26 (3) | 0 (0) | 0.3 (0.3) |
| TS Run-off Gush 2, g | 22 (3) | 0 (0) | 0 (0) |
| TS Run-off Gush 3, g | 19 (2) | 0 (0) | 0 (0) |
| TS Run-off Gush 4, g | 17 (3) | 0.5 (0.7) | 0.4 (0.8) |
| Total TS Run-off, g | 83 (3) | 0.5 (0.7) | 0.9 (1.1) |

As can be seen from Table 1 above, the second substrate had the least amount of run-off.

### Wet Length

**Table 2**

| The reported data is the average and the standard deviation is in parenthesis. | | | |
|---|---|---|---|
| | First Substrate on Swaddlers^{®} | Second Substrate on Swaddlers^{®} (*Present Disclosure*) | Third Substrate on Swaddlers^{®} |
| TS Run-off Gush 1, mm | 280 (0) | 124 (6) | 260 (40) |
| TS Run-off Gush 2, mm | 280 (0) | 153 (5) | 260 (40) |
| TS Run-off Gush 3, mm | 280 (0) | 186 (3) | 260 (40) |
| TS Run-off Gush 4, mm | 280 (0) | 280 (0) | 280 (0) |

As can be seen from Table 2, the second substrate had the least amount of wet length.

As it can be seen from both charts above, the data overall confirms the advantages of the present disclosure (second substrate) compared to both of the comparative examples (the first substrate and the second substrate) in terms of minimizing both run-off and wet length.

### First Substrate - Fully Hydrophobic Both Layers

The first substrate had two fully hydrophobic layers, without any hydrophilic material added proximate to the apertures. The first layer was a hydrophobic material made from PE/PET sheath/core (2 denier) staples fibers with a basis weight of 15 gsm. In a topsheet context, this first layer is the wearer-facing layer. The second layer was made from PE/PET sheath/core (2 denier) staple fibers with a basis weight of 20 gsm. In a topsheet context, this second layer is the garment-facing layer. The first layer was joined to the second through hot air bonding and then both layers were run through the rolls of Figs 27-29 as described herein. The z-directional height of the projections was in the first substrate was 1200µm. The recesses in the first substrate define a conical aperture at a location most distal from a top peak of an adjacent projection. The upper aperture diameter (about 1900µm) was larger than the bottom diameter (about 1500µm). The recesses had a z-directional height of about 500µm. The first substrate had an overall z-directional height of about 1700µm. The heights in this paragraph were measured according to the Height Tests herein

### Second Substrate-Both Layers Hydrophobic with Hydrophobic Material (Present Disclosure)

The second substrate is the same as the first substrate but with the hydrophilic material applied proximate to the apertures, as described with reference to Figs. 31-34.

### Third Substrate-Hydrophobic wearer-facing layer and Hydrophilic Garment-facing layer

The third substrate is the same as the first substrate, but the second layer is hydrophilic and made from PE/PET sheath/core (2 denier) staple fibers.

### Hydrophilic Substrates with Hydrophobic Materials

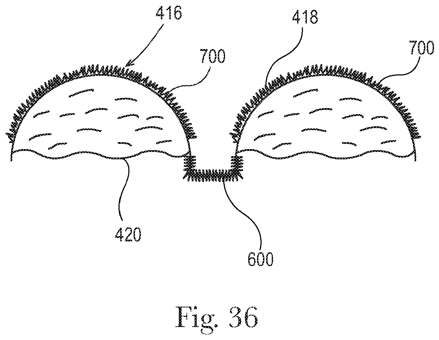

As a different form, the present disclosure may comprise a hydrophilic, nonwoven, apertured substrate. The substrate may have the same structure (projections, recesses, land areas etc.) as the three-dimensional, apertured substrates described herein, but may be hydrophilic instead of being hydrophobic. The substrate may have one or more hydrophilic layers. If more than one hydrophilic layer is provided, the various layers may be the same or different in denier, basis weight, fiber type etc., as described above with respect to the hydrophobic substrates with hydrophilic materials positioned only proximate to the apertures. For three-dimensional hydrophilic, apertured, substrates, the fluids tend to be quickly absorbed through/penetrate into the projections and land areas and also somewhat wick through the apertures 422. Referring to Fig. 36, in order to channel the fluids towards the recesses 414 and into and through the apertures 422, one or more hydrophobic materials 700 may be positioned on, or printed on, only the projections 416 and/or land areas. The hydrophobic material 700 being positioned on/printed on only the projections 416 and/or the land areas may cause the fluids to flow by gravity into the lower recesses 414, so that the fluids may be wicked through the apertures 422. The hydrophobic material 700 may prevent, or at least inhibit, the fluids from wicking through the projections and/or the land areas. The recesses 414 and the apertures 422 may be hydrophilic owing to the hydrophilicity of the substrates. In some instances, the hydrophilic materials 600 described above may also be applied only proximate to the apertures to further increase the hydrophilicity of areas proximate to the apertures 422 and/or portions of the recesses 414. In essence, this form of a hydrophilic substrate with one or more hydrophobic materials positioned on the projections and/or land areas accomplishes similar goals as the above described hydrophobic substrates with hydrophilic materials positioned only proximate to the apertures.

Some suitable examples of hydrophobic materials 700 comprise fluorinated or perfluorinated polymers; silicones; fluorochemicals; zirconium compounds; oils; latexes; waxes; crosslinking resins; and blends thereof; fluorochemical urethanes, ureas, esters, ethers, alcohols, epoxides, allophanates, amides, amines (and salts thereof), acids (and salts thereof), carbodiimides, guanidines, oxazolidinones, isocyanurates, and biurets; nanostructured particles selected from fumed silica, hydrophobic titania, zinc oxide, nanoclay, and mixtures thereof; fats and oils, glycerol derivatives; hydrophobic silicones or suitable combinations thereof.

Examples of suitable silicone polymers are selected from the group consisting of silicone MQ resins, polydimethysiloxanes, crosslinked silicones, silicone liquid elastomers, and combinations thereof. Polydimethylsiloxanes can be selected from the group consisting of vinyl-terminated polydimethsiloxanes, methyl hydrogen dimethylsiloxanes, hydroxyl-terminated polydimethysiloxanes, organo-modified polydimethylsiloxanes, and combinations thereof, among others.

Other hydrophobic materials 700 suitable for the present disclosure are well defined and documented in the art. For example, US Patent Application Publication No. 2002/0064639 A1 describes hydrophobic compositions selected from the group consisting of silicones, fluorochemicals, zirconium compounds, oils, latexes, waxes, crosslinking resins, and blends thereof. Representative water repellent fluorochemical compounds described in U.S. Pat. No. 7,407,899 include fluorochemical urethanes, ureas, esters, ethers, alcohols, epoxides, allophanates, amides, amines (and salts thereof), acids (and salts thereof), carbodiimides, guanidines, oxazolidinones, isocyanurates, and biurets. U.S. Pat. No. 6,548,732 describes hydrophobic substances from the group consisting of theobroma oil, cacao butter, cocoa butter, petrolatum, mineral jelly, white mineral oil, dimethicone, zinc oxide preparation, chinese white, zinc white, beeswax, lanolin, jojoba oil and combinations thereof. Additionally, U.S. application Ser. No. 13/193,065, filed Jul. 28, 2011 discusses substrates that exhibit superhydrophobic properties when treated with a composition comprising a hydrophobic component selected from fluorinated polymers, perfluorinated polymers, and mixtures thereof; nano-structured particles selected from fumed silica, hydrophobic titania, zinc oxide, nanoclay, and mixtures thereof; and water for an overall water-based, non-organic solvent. Examples of such compositions and surfaces in U.S. Pat. Application Ser. No. 13/193,065, filed on Jul. 28, 2011 exemplify the superhydrophobic treated surfaces that may be used as the nonwoven topsheet of the present disclosure.

Additionally waxes and other hydrophobic materials may be used, including petroleum-based emollients; fatty acid esters; polyol polyesters; fatty alcohol ethers; sterols and sterol esters, and their derivatives; triglycerides; glyceryl esters; ceramides; and mixtures thereof. The fatty acids may originate from vegetable, animal, and/or synthetic sources. Some fatty acids may range from a C8 fatty acid to a C30 fatty acid, or from a C12 fatty acid to a C22 fatty acid. In another embodiment, a substantially saturated fatty acid may be used, particularly when saturation arises as a result of hydrogenation of fatty acid precursor. Examples of fatty acid derivatives include fatty alcohols, fatty acid esters, and fatty acid amides.

Suitable fatty alcohols (R-OH) include those derived from C12-C28 fatty acids.
R-OH R=C12-C28 alkyl chain

Suitable fatty acid esters include those fatty acid esters derived from a mixture of C12-C28 fatty acids and short chain (C1-C8, preferably C1-C3) monohydric alcohols preferably from a mixture of C12-C22 saturated fatty acids and short chain (C1-C8, preferably C1-C3) monohydric alcohols. The hydrophobic melt additive may comprise a mixture of mono, di, and/or tri-fatty acid esters. An example includes fatty acid ester with glycerol as the backbone.

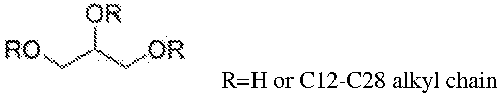

The glycerol derived fatty acid ester has at least one alkyl chain, at least two, or three chains to a glycerol, to form a mono, di, or triglyceride. Suitable examples of triglycerides include glycerol thibehenate (C22), glycerol tristearate (C18), glycerol tripalmitate (C16), and glycerol trimyristate (C14), and mixtures thereof. In the case of triglycerides and diglycerides, the alkyl chains could be the same length, or different length. Example includes a triglyceride with one alkyl C18 chain and two C16 alkyl chain, or two C18 alkyl chains and one C16 chain. Preferred triglycerides include alkyl chains derived from C14-C22 fatty acids.

Suitable fatty acid amides include those derives from a mixture of C12-C28 fatty acids (saturated or unsaturated) and primary or secondary amines. A suitable example of a primary fatty acid amide includes those derived from a fatty acid and ammonia.

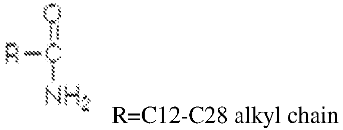

Suitable examples include erucamide, oleamide and behanamide. Other suitable hydrophobic melt additives include hydrophobic silicones, ethoxylated fatty alcohols.

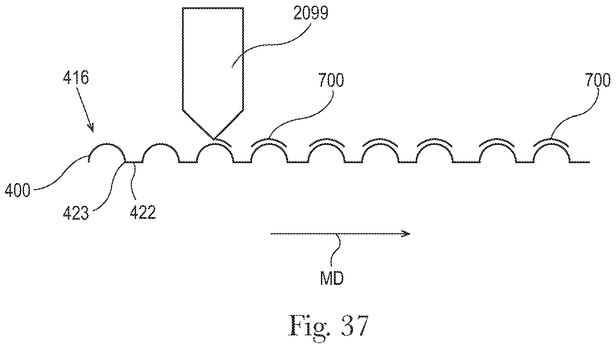

The hydrophobic materials 700 may be applied to the hydrophilic substrate using the ink jet printing methods described in U.S. Patent Application Serial No. 62/385,265, P&G Attorney Docket No. 14495PQ, filed on September 9, 2016. The hydrophobic materials 700 may also be applied using a modified process of Fig. 34. In this modified process, the substrate 400 may be flipped over so that the projections 416 and/or the land areas contact the roller 602. The hydrophilic material 600 may be replaced with the hydrophobic material 700. As such, the roller 602 can deposit the hydrophobic materials 700 on the projections and/or the land areas. The hydrophobic materials 700 may also be applied using a modified process of Fig. 35. The substrate 400 may be flipped over so that the projections 416 and/or land areas contact the flat clean surface 606. The hydrophilic material 600 may be replaced with the hydrophobic material 700. As such, the clean surface 606 can deposit the hydrophobic materials 700 on the projections and/or the land areas. In other instances, referring to Fig. 37, the hydrophilic substrate 400 may be conveyed in a machine direction, MD. A slot coater 2099 may be used to apply the hydrophobic material 700 only to the projections 416 and/or the land areas.

### TEST METHODS

Condition all samples at about 23 °C ± 2 C° and about 50% ± 2% relative humidity for 2 hours prior to testing.

### Aperture Test

Aperture dimensions, effective aperture area, and % effective open area measurements are performed on images generated using a flat bed scanner capable of scanning in reflectance mode at a resolution of 6400 dpi and 8 bit grayscale (a suitable scanner is the Epson Perfection V750 Pro, Epson, USA). Analyses are performed using ImageJ software (v.s 1.46, National Institute of Health, USA) and calibrated against a ruler certified by NIST. A steel frame (100 mm square, 1.5 mm thick with an opening 60 mm square) is used to mount the specimen and a black glass tile (P/N 11-0050-30, available from HunterLab, Reston, VA) is used as the background for the scanned images.

Take the steel frame and place double-sided adhesive tape on the bottom surface surrounding the interior opening. To obtain a specimen, lay the absorbent article flat on a lab bench with the wearer-facing surface directed upward. Remove the release paper of the tape, and adhere the steel frame to the topsheet (substrates described herein may only form a portion of the topsheet, e.g., by being positioned on the topsheet-the three-dimensional material is what is sampled) of the absorbent article. Using a razor blade, excise the top sheet from the underling layers of the absorbent article around the outer perimeter of the frame. Carefully remove the specimen such that its longitudinal and lateral extension is maintained. A cryogenic spray (such as Cyto-Freeze, Control Company, Houston TX) can be used to remove the topsheet specimen from the underling layers, if necessary. Five replicates obtained from five substantially similar absorbent articles are prepared for analysis.

Place the ruler on the scanner bed, close the lid and acquire a 50mm by 50mm calibration image of the ruler in reflectance mode at a resolution of 6400 dpi and 8 bit grayscale. Save the image as an uncompressed TIFF format file. Lift the lid and remove the ruler. After obtaining the calibration image, all specimens are scanned under the same conditions and measured based on the same calibration file. Next, place the framed specimen onto the center of the scanner bed with the wearer-facing surface of the specimen facing the scanner's glass surface. Place the black glass tile on top of the frame covering the specimen, close the lid and acquire a scanned image. In like fashion scan the remaining four replicates.

Open the calibration file in ImageJ and perform a linear calibration using the imaged ruler, with the scale set to Global so that the calibration will be applied to subsequent specimens. Open a specimen image in ImageJ. View the histogram and identify the gray level value for the minimum population located between the dark pixel peak of the holes and the lighter pixel peak of the nonwoven. Threshold the image at the minimum gray level value to generate a binary image. In the processed image, the apertures appear as black and nonwoven as white.

Select the analyze particles function. Set the minimum aperture area exclusion limit to 0.3 mm² and for the analysis to exclude the edge apertures. Set the software to calculate: effective aperture area, perimeter, feret (length of the aperture) and minimum feret (width of the aperture). Record the average effective aperture area to the nearest 0.01 mm², and the average perimeter to the nearest 0.01 mm. Again select the analyze particles function, but his time set the analysis to include the edge holes as it calculates the effective aperture areas. Sum the effective aperture areas (includes whole and partial apertures) and divide by the total area included in the image (2500 mm²). Record as the % effective open area to the nearest 0.01%.

In like fashion analyze the remaining four specimen images. Calculate and report the average effective aperture area to the nearest 0.01 mm², the average aperture perimeter to the nearest 0.01mm, feret and minimum feret to the nearest 0.01 mm, and the % effective open area to the nearest 0.01% for the five replicates.

If the apertures have a conical shape, effective aperture area is taken at the minimum dimension of the conical shape.

### Height Tests

Substrate projection heights and overall substrate heights are measured using a GFM MikroCAD Premium instrument commercially available from GFMesstechnik GmbH, Teltow/Berlin, Germany. The GFM MikroCAD Premium instrument includes the following main components: a) a DLP projector with direct digital controlled micro-mirrors; b) a CCD camera with at least a 1600 × 1200 pixel resolution; c) projection optics adapted to a measuring area of at least 60 mm × 45 mm; d) recording optics adapted to a measuring area of at least 60 mm × 45 mm; e) a table tripod based on a small hard stone plate; f) a blue LED light source; g) a measuring, control, and evaluation computer running ODSCAD software (version 6.2, or equivalent); and h) calibration plates for lateral (x-y) and vertical (z) calibration available from the vendor.

The GFM MikroCAD Premium system measures the surface height of a sample using the digital micro-mirror pattern fringe projection technique. The result of the analysis is a map of surface height (z-directional or z-axis) versus displacement in the x-y plane. The system has a field of view of 60 × 45 mm with an x-y pixel resolution of approximately 40 microns. The height resolution is set at 0.5 micron/count, with a height range of +/-15 mm. All testing is performed in a conditioned room maintained at about 23 ± 2 °C and about 50 ± 2 % relative humidity.

A steel frame (100 mm square, 1.5 mm thick with an opening 70 mm square) is used to mount the specimen. Take the steel frame and place double-sided adhesive tape on the bottom surface surrounding the interior opening. To obtain a specimen, lay the absorbent article flat on a bench with the wearer-facing surface directed upward. Remove the release paper of the tape, and adhere the steel frame to the topsheet (substrates described herein may only form a portion of the topsheet, e.g., by being positioned on the topsheet-the three-dimensional material is what is sampled) of the absorbent article. Using a razor blade, excise the topsheet from the underling layers of the absorbent article around the outer perimeter of the frame. Carefully remove the specimen such that its longitudinal and lateral extension is maintained. A cryogenic spray (such as Cyto-Freeze, Control Company, Houston TX) can be used to remove the topsheet specimen from the underling layers, if necessary. Five replicates obtained from five substantially similar absorbent articles are prepared for analysis.

Calibrate the instrument according to manufacturer's specifications using the calibration plates for lateral (x-y axis) and vertical (z axis) available from the vendor.

Place the steel plate and specimen on the table beneath the camera, with the wearer-facing surface oriented toward the camera. Center the specimen within the camera field of view, so that only the specimen surface is visible in the image. Allow the specimen to lay flat with minimal wrinkles.

Collect a height image (z-direction) of the specimen by following the instrument manufacturer's recommended measurement procedures. Select the Technical Surface/Standard measurement program with the following operating parameters: Utilization of fast picture recording with a 3 frame delay. Dual phase shifts are used with 1) 16 pixel stripe width with a picture count of 12 and 2) 32 pixel stripe width with a picture count of 8. A full Graycode starting with pixel 2 and ending with pixel 512. After selection of the measurement program, continue to follow the instrument manufacturer's recommended procedures for focusing the measurement system and performing the brightness adjustment. Perform the 3D measurement then save the height image and camera image files.

Load the height image into the analysis portion of the software via the clipboard. The following filtering procedure is then performed on each image: 1) removal of invalid points; 2) removal of peaks (small localized elevations); 3) polynomial filtering of the material part with a rank of n=5, with exclusion of 30% of the peaks and 30% of the valleys from the material part, and 5 cycles.

### Projection Height Test

Draw a line connecting the peaks of a series of projections, with the line crossing a non-apertured land area located between each of the projections. Generate a sectional image of the height image along the drawn line. Along the sectional line, measure the vertical height (z-direction) difference between the peak of the projection and the adjacent valley of the land area. Record the height to the nearest 0.1 µm. Average together 10 different projection peak to land area height measures and report this value to the nearest 0.1 µm. This is the projection height.

### Recess Height Test

Subtract the projection height from the overall substrate height to obtain the recess height. This should be done with each of the ten measurements from the Projection Height Test and the Overall Substrate Height Test. Average together the ten recess heights and report this value to the nearest 0.1 µm. This is the recess height.

### Overall Substrate Height Test

Draw a line connecting the peaks of a series of projections, with the line crossing the center of an aperture located between each of the projections and within a recess. Generate a sectional image of the height image along the drawn line. Along the sectional line, measure the vertical height difference between the peak of the projection and the adjacent base of the recess. Record the height to the nearest 0.1 µm. Average together 10 different projection peak to base of recess height measures and report this value to the nearest 0.1 µm. This is the overall substrate height.

### Average Aperture Spacing Test

Lateral Axis Aperture Spacing and Longitudinal Axis Aperture Spacing are performed on images generated using a flat bed scanner capable of scanning in reflectance mode at a resolution of 6400 dpi and 8 bit grayscale (a suitable scanner is the Epson Perfection V750 Pro, Epson, USA). Analyses are performed using ImageJ software (v.s 1.46, National Institute of Health, USA) and calibrated against a ruler certified by NIST. A steel frame (100 mm square, 1.5 mm thick with an opening 60 mm square) is used to mount the specimen and a black glass tile (P/N 11-0050-30, available from HunterLab, Reston, VA) is used as the background for the scanned images. Testing is performed at about 23 °C ± 2 C° and about 50% ± 2% relative humidity.

Take the steel frame and place double-sided adhesive tape on the bottom surface surrounding the interior opening. To obtain a specimen, lay the absorbent article flat on a lab bench with the wearer-facing surface directed upward. Remove the release paper of the tape, and adhere the steel frame to the topsheet of the absorbent article. Using a razor blade excise the topsheet (i.e., the three dimensional substrate that forms all of or part of the wearer-facing surface) from the underling layers of the absorbent article around the outer perimeter of the frame. Carefully remove the specimen such that its longitudinal and lateral extension is maintained. A cryogenic spray (such as Cyto-Freeze, Control Company, Houston TX) can be used to remove the topsheet specimen from the underling layers, if necessary. Five replicates obtained from five substantially similar absorbent articles are prepared for analysis. Condition the samples at about 23 °C ± 2 C° and about 50% ± 2% relative humidity for 2 hours prior to testing.

Place the ruler on the scanner bed, close the lid and acquire a 50mm by 50mm calibration image of the ruler in reflectance mode at a resolution of 6400 dpi and 8 bit grayscale. Save the image as an uncompressed TIFF format file. Lift the lid and remove the ruler. After obtaining the calibration image, all specimens are scanned under the same conditions and measured based on the same calibration file. Next, place the framed specimen onto the center of the scanner bed with the wearer-facing surface of the specimen facing the scanner's glass surface. Place the black glass tile on top of the frame covering the specimen, close the lid and acquire a scanned image. In a like fashion, scan the remaining four replicates.

Open the calibration file in ImageJ and perform a linear calibration using the imaged ruler, with the scale set to Global so that the calibration will be applied to subsequent specimens. Open a specimen image in ImageJ and perform the following measures:

### Lateral Axis Aperture Spacing

Measure from a center point of one aperture to a center point of an adjacent aperture on the other side of a projection, wherein the projection is positioned between the two apertures. The measurement will be taken in a direction parallel to a lateral axis of the specimen across the projection. Report each distance to the nearest 0.1mm. Take 5 random measurements in the specimen. Average the five values to and report the average lateral axis center to center spacing to the nearest 0.1mm. Repeat this procedure for the additional four samples.

### Longitudinal Axis Aperture Spacing

Measure from a center point of one aperture to a center point of an adjacent aperture on the other side of a projection, wherein the projection is positioned between the two apertures. The measurement will be taken in a direction parallel to a longitudinal axis of the specimen across the projection. Report each distance to the nearest 0.1mm. Take 5 random measurements in the specimen. Average the five values to and report the average longitudinal axis center to center spacing to the nearest 0.1mm. Repeat this procedure for the additional four samples.

### Basis Weight Test

Basis weight of the three-dimensional substrates may be determined by several available techniques but a simple representative technique involves taking an absorbent article, removing any elastic which may be present and stretching the absorbent article to its full length. A punch die having an area of 45.6 cm² is then used to cut a piece of the substrate forming a topsheet, positioned on the topsheet, or forming a portion of the topsheet (the "topsheet" in this method), from the approximate center of the diaper or absorbent product in a location which avoids to the greatest extent possible any adhesive which may be used to fasten the topsheet to any other layers which may be present and removing the topsheet layer from other layers (using cryogenic spray, such as Cyto-Freeze, Control Company, Houston, Tx. if needed). The sample is then weighed and dividing by the area of the punch die yields the basis weight of the topsheet. Results are reported as a mean of 5 samples to the nearest 0.1 gram per square meter.

### In-Bag Stack Height Test

The in-bag stack height of a package of absorbent articles is determined as follows:

### Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

### Test Procedure

Absorbent article packages are equilibrated at 23 ± 2 °C and 50 ± 5 % relative humidity prior to measurement.

The horizontal sliding plate is raised and an absorbent article package is placed centrally under the horizontal sliding plate in such a way that the absorbent articles within the package are in a horizontal orientation (see Fig. 30). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

### Run-Off Test

The Run-off Test is intended to test whether the test liquid gets absorbed quickly enough into the diaper as opposed to running-off over the surface of the topsheet and leaking at the back edge of the diaper. This method determines the liquid run-off of a baby diaper typically designated for infants, e.g. wearers having a weight in the range of 5 to 8 kg ± 20% (such as Pampers baby diapers size 2 or baby diapers size 2 or size S of most other tradenames). All testing is carried out at 23 ± 2°C and 40-55% relative humidity.

The test apparatus comprises an angled table made of polycarbonate (e.g. Lexan^{®}) about 10 mm in thickness. The angled table is 520mm long and 160mm wide and is inclined by 30±1 degrees vs. horizontal: the dimensions of the table can be adjusted such to be larger than the diaper dimensions. The angled table is attached to a support, made of polycarbonate (e.g. Lexan^{®}) about 10 mm in thickness or an equivalent suitable material.

A diaper is removed from its packaging and the leg cuff elastics are cut at suitable intervals to allow the diaper to lay flat: it must be taken care to touch the topsheet as little as possible and not to damage the diaper core. The product is weighed to within ± 0.1 grams on a suitable top-loading balance then attached via bi-adhesive tape onto the angled table such that the product is centered along the longitudinal centerline of the apparatus with the topsheet (body-side) of the product facing upwards, its front waist edge standing on the upper part of the angled table and its back edge being in line with the lower edge of the angled table: the loading point is marked onto the product via a water resistant pen at a position 76 mm from the front core absorbent edge.

A suitable pump; e.g. Model 7520-00 supplied by Cole Parmer Instruments, Chicago, USA, or equivalent; is set up to discharge a 0.9 mass % aqueous solution of sodium chloride through a flexible plastic tube having an internal diameter of 4.8 mm, e.g., Tygon^{®} R-3603 or equivalent. The end portion of the tube is clamped vertically so that it is centered vertically 25mm above the loading point marked on the product: the position of the end portion of the tube is adjusted after each gush to maintain the distance of 25mm above the loading point marked on the product. The pump is operated via a timer and is pre-calibrated to discharge a gush of 40.0 ml of the 0.9% saline solution at a rate of 10 ml/sec. Four gushes are delivered to the product in this fashion; the time interval between the beginning of a certain gush and the beginning of the next gush is 300 seconds.

A suitable Petri dish is pre-weighted and placed under the lower edge of the angled table to collect the run-off liquid. The test liquid run-off is calculated after each gush via subtracting the weight of the Petri dish with run-off liquid and the dry weight of the Petri dish. The total test liquid run-off is also reported and calculated as the sum of the first, second, third and fourth gush run-off. After each gush a dry Petri dish is used. Four products for each option are tested in this fashion and the average for each of the respective gushes (first through fourth) and for the total run-off is calculated.

"Wet length" is the distance of the liquid running down on the topsheet from the first liquid contact to topsheet (loading point) until the area where the liquid is absorbed. The "wet length" is measured in parallel to a central longitudinal axis of the topsheet (a straight line) from the loading point to the location of the area where the liquid is absorbed. The "wet length" is measured 60 seconds after every gush. A blue pigment (Indigo Carmine (C.I.73015)) can be added to the liquid for better visualization of the absorbency area to determine the wet length. Specifically for the examples shown herein the max distance from the pee point to the diaper edge is 280mm.

## Claims

1. A liquid permeable nonwoven topsheet (24) for an absorbent article (20), the topsheet (24) comprising: a nonwoven, hydrophobic material; and
wherein the topsheet (24) comprises a plurality of recesses (414), a plurality of projections (416), and a plurality of land areas (412), wherein the land areas surround at least a majority of the plurality of projections (416) and a plurality of the recesses (414), wherein the plurality of recesses (414), the plurality of projections (416), and the plurality of land areas (412), together form a first three-dimensional surface on a first side of the topsheet (24) and a second three-dimensional surface on a second side of the topsheet (24), wherein a majority of the projections (416) have a z-directional height in the range of about 400µm to about 4000µm, according to the Projection Height Test as set out herein, wherein a majority of the recesses (414) define an aperture at a location most distal from a top peak of an adjacent projection, and wherein the majority of the recesses (414) have a z-directional height in the range of about 400µm to about 2000µm, according to the Recess Height Test as set out herein;
wherein the substrate (400) has an overall z-directional height in the range of about 800µm to about 6000µm, according to the Overall Substrate Height Test as set out herein; and
wherein a hydrophilic material is positioned only proximate to at least some of the apertures in that it is provided only around perimeters of the apertures, only in areas closely surrounding the apertures, and/or only around perimeters of the apertures and in portions of the recesses; and
wherein the nonwoven, hydrophobic material comprises:
a first nonwoven, hydrophobic layer;
a second nonwoven, hydrophobic layer, wherein the first layer is joined to the second layer, and wherein the first layer has a different basis weight than the second nonwoven;
wherein a portion of the projections (416) and a portion of the recesses (414) are formed by a portion of the first layer and a portion of the second layer; and
wherein the apertures are formed through the first layer and through the second layer; and
wherein the projections (416) and land areas (412) are hydrophobic.

2. The topsheet (24) according to Claim 1, wherein the recesses (414) comprises the hydrophilic material in areas around perimeters of the apertures.

3. The topsheet (24) according to any one of the preceding claims, wherein a majority of the apertures (422) have an effective aperture area in the range of about 1.0 mm² to about 3.5 mm², according to the Aperture Test as set out herein.

4. The topsheet (24) according to any one of the preceding claims, wherein the topsheet (24) has a % effective open area in the range of about 5% to about 20%, according to the Aperture Test as set out herein.

5. The topsheet (24) according to any one of the preceding claims, wherein the first layer is joined to the second layer by passing heated air through the first layer and the second layer.

6. The topsheet (24) according to any one of the preceding claims, wherein the first layer comprises a plurality of first fibers, wherein the second layer comprises a plurality of second fibers, and wherein the first and second fibers are different.

7. The topsheet (24) according to any one of the preceding claims, wherein four apertures are formed around each projection, and wherein four projections are formed around each aperture.

8. The topsheet (24) according to any one of the preceding claims, wherein two adjacent apertures are separated by a projection (416) and a land area (412) along a lateral axis (90) of the substrate (400), wherein two adjacent projections are separated by an aperture and a land area (412) along the lateral axis (90) of the substrate (400), wherein two adjacent apertures are separated by a projection (416) and a land area (412) along a longitudinal axis (80) of the substrate (400), and wherein two adjacent projections are separated by an aperture and a land area (412) along the longitudinal axis (80) of the substrate (400).

9. The topsheet (24) according to any one of the preceding claims, wherein substantially all of the recesses (414) define an aperture, and wherein substantially all of the projections (416) comprise a hollow arched portion.

10. The topsheet (24) according to any one of the preceding claims, wherein the apertures comprise a first set of apertures together forming a first line in the substrate (400) and a second set of apertures together forming a second line in the substrate (400), and wherein the first line is generally parallel with the second line.

11. The topsheet (24) according to any one of the preceding claims, wherein a perimeter of the majority of the apertures (422) forms a first plane of the bottommost portion of the substrate (400), wherein a top peak of the majority of the projections (416) forms a second plane of the topmost portion of the substrate (400), and wherein the land areas (412) are positioned intermediate the first plane and the second plane.

12. The topsheet (24) according to any one of the preceding claims, wherein the first layer comprises fibers that are at least 0.5 denier greater than the denier of the fibers of the second layer.

13. The topsheet (24) according to any one of the preceding claims, wherein the first layer comprises fibers having a denier in the range of about 1.2 to about 4, and wherein the second layer comprises fibers having a denier in the range of about 1.0 to about 3.5.

14. An absorbent article (20) comprising:
a topsheet (24) according to any one of the preceding claims;
a backsheet (25); and
an absorbent core (28) positioned at least partially intermediate the topsheet (24) and the backsheet (25).

15. The absorbent article (20) according to Claim 14, wherein the absorbent core (28) comprises an absorbent material comprising at least 90% superabsorbent polymers by weight of the absorbent material.

## Patentansprüche

1. Flüssigkeitsdurchlässige Vliesoberschicht (24) für einen Absorptionsartikel, die Oberschicht (24) umfassend: ein hydrophobes Vliesmaterial; und
wobei die Oberschicht (24) eine Vielzahl von Vertiefungen (414), eine Vielzahl von Vorsprüngen (416) und eine Vielzahl von Auftreffbereichen (412) umfasst, wobei die Auftreffbereiche wenigstens einen Großteil der Vielzahl von Vorsprüngen (416) und eine Vielzahl der Vertiefungen (414) umgeben, wobei die Vielzahl von Vertiefungen (414), die Vielzahl von Vorsprüngen (416) und die Vielzahl von Auftreffbereichen (412) zusammen eine erste dreidimensionale Oberfläche auf einer ersten Seite der Oberschicht (24) und eine zweite dreidimensionale Oberfläche auf einer zweiten Seite der Oberschicht (24) ausbilden, wobei ein Großteil der Vorsprünge (416) nach der hierin beschriebenen Vorsprungshöhenprüfung eine z-gerichtete Höhe im Bereich von etwa 400 µm bis etwa 4000 µm aufweist, wobei ein Großteil der Vertiefungen (414) eine Öffnung an einer Stelle bestimmt, die am distalsten von einer oberen Spitze eines benachbarten Vorsprungs entfernt ist, und wobei der Großteil der Vertiefungen (414) nach der hierin beschriebenen Vertiefungshöhenprüfung eine z-gerichtete Höhe im Bereich von etwa 400 µm bis etwa 2000 µm aufweist;
wobei das Substrat (400) nach der hierin beschriebenen Substratgesamthöhenprüfung eine z-gerichtete Gesamthöhe im Bereich von etwa 800 µm bis etwa 6000 µm aufweist; und
wobei ein hydrophiles Material nur nahe wenigstens einiger der Öffnungen angeordnet ist, dadurch, dass es nur um Umfänge der Öffnungen, nur in Flächen, die die Öffnungen eng umgeben, und/oder nur um Umfänge der Öffnungen und in Abschnitten der Vertiefungen bereitgestellt wird; und
wobei das hydrophobe Vliesmaterial umfasst:
eine erste hydrophobe Vliesschicht;
eine zweite hydrophobe Vliesschicht, wobei die erste Schicht mit der zweiten Schicht gefaltet ist, und wobei die erste Schicht ein unterschiedliches Flächengewicht als das zweite Vlies aufweist;
wobei ein Abschnitt der Vorsprünge (416) und ein Abschnitt der Vertiefungen (414) durch einen Abschnitt der ersten Schicht und einen Abschnitt der zweiten Schicht ausgebildet sind; und
wobei die Öffnungen durch die erste Schicht und durch die zweite Schicht ausgebildet sind; und
wobei die Vorsprünge (416) und die Auftreffbereiche (412) hydrophob sind.

2. Oberschicht (24) nach Anspruch 1, wobei die Vertiefungen (414) das hydrophile Material in Flächen um Umfänge der Öffnungen umfassen.

3. Oberschicht (24) nach einem der vorstehenden Ansprüche, wobei ein Großteil der Öffnungen (422) nach der hierin beschriebenen Öffnungsprüfung eine wirksame Öffnungsfläche im Bereich von etwa 1,0 mm² bis etwa 3,5 mm² aufweist.

4. Oberschicht (24) nach einem der vorstehenden Ansprüche, wobei die Oberschicht (24) nach der hierin beschriebenen Öffnungsprüfung einen % wirksame offene Fläche im Bereich von etwa 5 % bis etwa 20 % aufweist.

5. Oberschicht (24) nach einem der vorstehenden Ansprüche, wobei die erste Schicht mit der zweiten Schicht durch Durchlassen von Warmluft durch die erste Schicht und die zweite Schicht gefaltet wird.

6. Oberschicht (24) nach einem der vorstehenden Ansprüche, wobei die erste Schicht eine Vielzahl von ersten Fasern umfasst, wobei die zweite Schicht eine Vielzahl von zweiten Fasern umfasst, und wobei die ersten und die zweiten Fasern unterschiedlich sind.

7. Oberschicht (24) nach einem der vorstehenden Ansprüche, wobei vier Öffnungen um jeden Vorsprung herum ausgebildet sind, und wobei vier Vorsprünge um jede Öffnung herum ausgebildet sind.

8. Oberschicht (24) nach einem der vorstehenden Ansprüche, wobei zwei benachbarte Öffnungen durch einen Vorsprung (416) und einen Auftreffbereich (412) entlang einer Querachse (90) des Substrats (400) getrennt sind, wobei zwei benachbarte Vorsprünge durch eine Öffnung und einen Auftreffbereich (412) entlang der Querachse (90) des Substrats (400) getrennt sind, wobei zwei benachbarte Öffnungen durch einen Vorsprung (416) und einen Auftreffbereich (412) entlang einer Längsachse (80) des Substrats (400) getrennt sind, und wobei zwei benachbarte Vorsprünge durch eine Öffnung und einen Auftreffbereich (412) entlang der Längsachse (80) des Substrats (400) getrennt sind.

9. Oberschicht (24) nach einem der vorstehenden Ansprüche, wobei im Wesentlichen sämtliche Vertiefungen (414) eine Öffnung bestimmen, und wobei im Wesentlichen sämtliche Vorsprünge (416) einen hohlen gewölbten Abschnitt umfassen.

10. Oberschicht (24) nach einem der vorstehenden Ansprüche, wobei die Öffnungen einen ersten Satz von Öffnungen, die zusammen eine erste Linie in dem Substrat (400) ausbilden, und einen zweiten Satz von Öffnungen, die zusammen eine zweite Linie in dem Substrat (400) ausbilden, umfassen, und wobei die erste Linie im Allgemeinen parallel zu der zweiten Linie verläuft.

11. Oberschicht (24) nach einem der vorstehenden Ansprüche, wobei ein Umfang des Großteils der Öffnungen (422) eine erste Ebene des untersten Abschnitts des Substrats (400) ausbildet, wobei eine obere Spitze des Großteils der Vorsprünge (416) eine zweite Ebene des obersten Abschnitts des Substrats (400) ausbildet, und wobei die Auftreffbereiche (412) zwischen der ersten Ebene und der zweiten Ebene angeordnet sind.

12. Oberschicht (24) nach einem der vorstehenden Ansprüche, wobei die erste Schicht Fasern umfasst, die wenigstens 0,5 Denier größer als das Denier der Fasern der zweiten Schicht sind.

13. Oberschicht (24) nach einem der vorstehenden Ansprüche, wobei die erste Schicht Fasern umfasst, die ein Denier im Bereich von etwa 1,2 bis etwa 4 aufweisen, und wobei die zweite Schicht Fasern umfasst, die ein Denier im Bereich von etwa 1,0 bis etwa 3,5 aufweisen.

14. Absorptionsartikel (20), umfassend:
eine Oberschicht(24) nach einem der vorstehenden Ansprüche;
eine Unterschicht (25); und
einen Absorptionskern (28), der wenigstens teilweise zwischen der Oberschicht (24) und der Unterschicht (25) angeordnet ist.

15. Absorptionsartikel (20) nach Anspruch 14, wobei der Absorptionskern (28) ein Absorptionsmaterial umfasst, umfassend wenigstens 90 % Superabsorber-Polymere bezogen auf das Gewicht des Absorptionsmaterials.

## Revendications

1. Feuille supérieure non tissée perméable aux liquides (24) pour un article absorbant, la feuille supérieure (24) comprenant : un matériau hydrophobe non tissé ; et
la feuille supérieure (24) comprenant une pluralité d'évidements (414), une pluralité de saillies (416), et une pluralité de surfaces d'appui (412), les surfaces d'appui entourant au moins une majorité de la pluralité de saillies (416) et une pluralité des évidements (414), la pluralité d'évidements (414), la pluralité de saillies (416), et la pluralité de surfaces d'appui (412), formant ensemble une première surface tridimensionnelle sur un premier côté de la feuille supérieure (24) et une seconde surface tridimensionnelle sur un second côté de la feuille supérieure (24), une majorité des saillies (416) ayant une hauteur de direction z dans la plage d'environ 400 µm à environ 4 000 µm, selon le test de hauteur de saillie, une majorité des évidements (414) définissant une ouverture à un emplacement le plus distal d'un sommet supérieur d'une saillie adjacente, et la majorité des évidements (414) ayant une hauteur de direction z dans la plage d'environ 400 µm à environ 2 000 µm, selon le test de hauteur d'évidement tel que défini ici ;
la feuille supérieure (400) ayant une hauteur de direction z globale dans la plage d'environ 800 µm à environ 6 000 µm, selon le test de hauteur globale de substrat tel que défini ici ; et
un matériau hydrophile étant positionné uniquement à proximité d'au moins certaines des ouvertures en ce qu'il est fourni uniquement autour des périmètres des ouvertures, uniquement dans des zones entourant étroitement les ouvertures, et/ou uniquement autour des périmètres des ouvertures et dans une partie des évidements ; et
le matériau hydrophobe non tissé comprenant :
une première couche hydrophobe non tissée ;
une seconde couche hydrophobe non tissée, la première couche étant liée à la seconde couche, et la première couche ayant une masse surfacique différente de la seconde non tissée ;
une partie des saillies (416) et une partie des évidements (414) étant formées par une partie de la première couche et une partie de la seconde couche ; et
les ouvertures étant formées à travers la première couche et à travers la seconde couche ; et
les saillies (416) et les surfaces d'appui (412) étant hydrophobes.

2. Feuille supérieure (24) selon la revendication 1, les évidements (414) comprenant le matériau hydrophile dans des zones autour des périmètres des ouvertures.

3. Feuille supérieure (24) selon l'une quelconque des revendications précédentes, une majorité des ouvertures (422) ayant une aire d'ouverture efficace dans la plage d'environ 1,0 mm² à environ 3,5 mm², selon le test d'ouverture tel que défini ici.

4. Feuille supérieure (24) selon l'une quelconque des revendications précédentes, la feuille supérieure (24) ayant une aire ouverte effective dans la plage d'environ 5 % à environ 20 %, selon le test d'ouverture tel que défini ici.

5. Feuille supérieure (24) selon l'une quelconque des revendications précédentes, la première couche étant liée à la seconde couche en faisant passer de l'air chauffé à travers la première couche et la seconde couche.

6. Feuille supérieure (24) selon l'une quelconque des revendications précédentes, la première couche comprenant une pluralité de premières fibres, la seconde couche comprenant une pluralité de secondes fibres, et les premières et secondes fibres étant différentes.

7. Feuille supérieure (24) selon l'une quelconque des revendications précédentes, quatre ouvertures étant formées autour de chaque saillie, et quatre saillies étant formées autour de chaque ouverture.

8. Feuille supérieure (24) selon l'une quelconque des revendications précédentes, deux ouvertures adjacentes étant séparées par une saillie (416) et une surface d'appui (412) le long d'un axe latéral (90) du substrat (400), deux saillies adjacentes étant séparées par une ouverture et une surface d'appui (412) le long de l'axe latéral (90) du substrat (400), deux ouvertures adjacentes étant séparées par une saillie (416) et une surface d'appui (412) le long d'un axe longitudinal (80) du substrat (400), et deux saillies adjacentes étant séparées par une ouverture et une surface d'appui (412) le long de l'axe longitudinal (80) du substrat (400).

9. Feuille supérieure (24) selon l'une quelconque des revendications précédentes, essentiellement tous les évidements (414) définissant une ouverture, et essentiellement toutes les saillies (416) comprenant une partie arquée creuse.

10. Feuille supérieure (24) selon l'une quelconque des revendications précédentes, les ouvertures comprenant un premier ensemble d'ouvertures formant ensemble une première ligne dans le substrat (400) et un second ensemble d'ouvertures formant ensemble une seconde ligne dans le substrat (400), et la première ligne étant généralement parallèle à la seconde ligne.

11. Feuille supérieure (24) selon l'une quelconque des revendications précédentes, un périmètre de la majorité des ouvertures (422) formant un premier plan de la partie la plus basse du substrat (400), un sommet supérieur de la majorité des saillies (416) formant un second plan de la partie la plus haute du substrat (400), et les surfaces d'appui (412) étant positionnées entre le premier plan et le second plan.

12. Feuille supérieure (24) selon l'une quelconque des revendications précédentes, la première couche comprenant des fibres qui sont supérieures d'au moins 0,5 denier au denier des fibres de la seconde couche.

13. Feuille supérieure (24) selon l'une quelconque des revendications précédentes, la première couche comprenant des fibres ayant un denier compris dans la plage d'environ 1,2 à environ 4, et la seconde couche des fibres ayant un denier compris dans la plage d'environ 1,0 à environ 3,5.

14. Article absorbant (20) comprenant :
une feuille supérieure (24) selon l'une quelconque des revendications précédentes ;
une feuille de fond (25) ; et
une âme absorbante (28) positionnée au moins partiellement entre la feuille supérieure (24) et la feuille de fond (25).

15. Article absorbant (20) selon la revendication 14, l'âme absorbante (28) comprenant un matériau absorbant comprenant au moins 90 % de polymères superabsorbants en poids du matériau absorbant.
